(19) Europäisches Patentamt
European Patent Office
Office européen des brevets

(11) **EP 1 140 187 B1**

(12) **EUROPEAN PATENT SPECIFICATION**

(45) Date of publication and mention of the grant of the patent:
**03.09.2003 Bulletin 2003/36**

(21) Application number: **99965901.4**

(22) Date of filing: **17.12.1999**

(51) Int Cl.[7]: **A61K 45/06**, A61K 31/55, A61K 31/585, A61P 9/00, A61K 31/575

(86) International application number:
**PCT/US99/27946**

(87) International publication number:
**WO 00/038725 (06.07.2000 Gazette 2000/27)**

(54) **COMBINATIONS OF AN IBAT INHIBITOR AND A MTP INHIBITOR FOR CARDIOVASCULAR INDICATIONS**

KOMBINATIONEN EINEM IBAT INHIBITOR UND EINEM MTP INHIBITOR FÜR KARDIOVASKULÄRE INDIKATIONEN

COMBINAISONS D'UN INHIBITEUR D'IBAT ET D'UN INHIBITEUR DE MTP UTILISEES DANS LE CADRE DE TROUBLES CARDIO-VASCULAIRES

(84) Designated Contracting States:
**AT BE CH CY DE DK ES FI FR GB GR IE IT LI LU MC NL PT SE**
Designated Extension States:
**RO**

(30) Priority: **23.12.1998 US 113955 P**

(43) Date of publication of application:
**10.10.2001 Bulletin 2001/41**

(73) Proprietor: **G.D. Searle LLC**
**Chicago, Illinois 60680-5110 (US)**

(72) Inventors:
- **KELLER, Bradley, T.**
  **Chesterfield, MO 63017 (US)**
- **REITZ, David, B.**
  **Chesterfield, MO 63017 (US)**
- **SCHUH, Joseph, R.**
  **St. Louis, MO 63146 (US)**
- **SIKORSKI, James, A.**
  **St. Louis, MO 63131 (US)**
- **TREMONT, Samuel, J.**
  **St. Louis, MO 63011 (US)**
- **LAPPE, Rodney, W.**
  **Chesterfield, MO 63005 (US)**

(74) Representative:
**Weisert, Annekäte, Dipl.-Ing. Dr.-Ing.**
**Patentanwälte**
**Kraus & Weisert**
**Thomas-Wimmer-Ring 15**
**80539 München (DE)**

(56) References cited:
**WO-A-94/09774** **WO-A-98/23593**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 1 140 187 B1

## Description

## BACKGROUND OF THE INVENTION

### Field of the Invention

The present invention relates to composition for treating cardiovascular diseases, and specifically relates to combinations of compounds, compositions, and their use in medicine, particularly in the prophylaxis and treatment of hyperlipidemic conditions such as are associated with atherosclerosis, hypercholesterolemia, and other factors in coronary artery disease in mammals including hypertension. More particularly, the invention relates to ileal bile acid transporter (IBAT) inhibitors, and microsomal triglyceride transfer.protein (MTP) inhibitors.

### Description of Related Art

It is well-settled that hyperlipidemic conditions associated with elevated concentrations of total cholesterol and low-density lipoprotein (LDL). cholesterol are major risk factors for coronary heart disease and particularly atherosclerosis. Numerous studies have demonstrated that a low plasma concentration of high density lipoprotein (HDL) cholesterol is a powerful risk factor for the development of atherosclerosis (Barter and Rye, Atherosclerosis, 121, 1-12 (1996)). HDL is one of the major classes of lipoproteins that function in the .transport of lipids through the blood. The major lipids found associated with HDL include cholesterol, cholesteryl ester, triglycerides, phospholipids and fatty acids. The other classes of lipoproteins found in the blood are low density lipoprotein (LDL), intermediate density lipoprotein (IDL), and very low density lipoprotein (VLDL). Since low levels of HDL cholesterol increase the risk of atherosclerosis, methods for elevating plasma HDL cholesterol would be therapeutically beneficial for the treatment of atherosclerosis and other diseases associated with accumulation of lipid in the blood vessels. These diseases include, but are not limited to, coronary heart disease, peripheral vascular disease, and stroke.

Atherosclerosis underlies most coronary artery disease (CAD), a major cause of morbidity and mortality in modern society. High LDL cholesterol (above about 180 mg/dl) and low HDL cholesterol (below 35 mg/dl) have been shown to be important contributors to the development of atherosclerosis. Other diseases or risk factors, such as peripheral vascular disease, stroke, and hypercholesterolaemia are negatively affected by adverse HDL/LDL ratios.

Interfering with the recirculation of bile acids from the lumen of the intestinal tract is found to reduce the levels of serum cholesterol in a causal relationship. Epidemiological data has accumulated which indicates such reduction leads to an improvement in the disease state of atherosclerosis. Stedronsky, in "Interaction of bile acids and cholesterol with nonsystemic agents having hypocholesterolemic properties," Biochimica et Biophysica Acta, 1210, 255-287 (1994) discusses the biochemistry, physiology and known active agents surrounding bile acids and cholesterol.

Transient pathophysiologic alterations are shown to be consistent with interruption of the enterohepatic circulation of bile acids in humans with an inherited lack of IBAT activity, as reported by Heubi, J.E., et al. See "Primary Bile Acid Malabsorption: Defective in Vitro Ileal Active Bile Acid Transport", Gastroenterology, 83, 804-11 (1982).

In another approach to the reduction of recirculation of bile acids, the ileal bile acid transport system is a putative pharmaceutical target for the treatment of hypercholesterolemia based on an interruption of the enterohepatic circulation with specific transport inhibitors (Kramer, et al., "Intestinal Bile Acid Absorption" The Journal of Biological Chemistry, 268 (24), 18035-46 (1993).

In several individual patent applications, Hoechst Aktiengesellschaft discloses polymers of various naturally occurring constituents of the enterohepatic circulation system and their derivatives, including bile acid, which inhibit the physiological bile acid transport with the goal of reducing the LDL cholesterol level sufficiently to be effective as pharmaceuticals and, in particular for use as hypocholesterolemic agents. The individual Hoechst patent applications which disclose such bile acid transport inhibiting compounds are each separately listed below.

R1. Canadian Patent Application No. 2,025,294.
R2. Canadian Patent Application No. 2,078,588.
R3. Canadian Patent Application No. 2,085,782.
R4. Canadian Patent Application No. 2,085,830.
R5. EP Application No. 0 379 161.
R6. EP Application No. 0 549 967.
R7. EP Application No. 0 559 064.
R8. EP Application No. 0 563 731.

Selected benzothiepines are disclosed in world patent application number WO 93/321146 for numerous uses including fatty acid metabolism and coronary vascular diseases.

Other selected benzothiepines are known for use as hypolipaemic and hypocholesterolaemic agents, especially for the treatment or prevention of atherosclerosis as disclosed in application No. EP 508425. A French patent application, FR 2661676 discloses additional benzothiepines for use as hypolipaemic and hypocholesterolaemic agents. Furthermore, patent application no. WO 92/18462 lists other benzothiepines for use as hypolipaemic and hypocholesterolaemic agents. U.S. Patent No. 5,994,391 (Lee et al.) Each of the benzothiepine hypolipaemic and hypocholesterolaemic agents described in these individual patent applications is limited by an amide bonded to the carbon adjacent the phenyl ring of the fused bicyclobenzothiepine ring.

Further benzothiepines useful for the treatment of hypercholesterolemia and hyperlipidemia are disclosed in patent application no. PCT/US95/10863. More benzothiepines useful for the prophylaxis and treatment of hypercholesterolemia and hyperlipidemia as well as pharmaceutical compositions of such benzothiepines are described in PCT/US97/04076. Still further benzothiepines and compositions thereof useful for the prophylaxis and treatment of hypercholesterolemia and hyperlipidemia are described in U.S. Application Serial No. 08/816,065.

In vitro bile acid transport inhibition is disclosed to correlate with hypolipidemic activity in The Wellcome Foundation Limited disclosure of the Patent Application No. WO 93/16055 for "Hypolipidemic Benzothiazepine Compounds." That publication describes a number of hypolipidemic benzothiazepine compounds. Additional hypolipidemic benzothiazepine compounds (particularly 2,3,4,5-tetrahydrobenzo-1-thi-4-azepine compounds) are disclosed in Patent Application No. WO 96/05188. A particularly useful benzothiazepine disclosed in WO 96/05188 is the compound of formula B-2. Further hypolipidemic benzothiazepine compounds are described in Patent Application No. WO 96/16051.

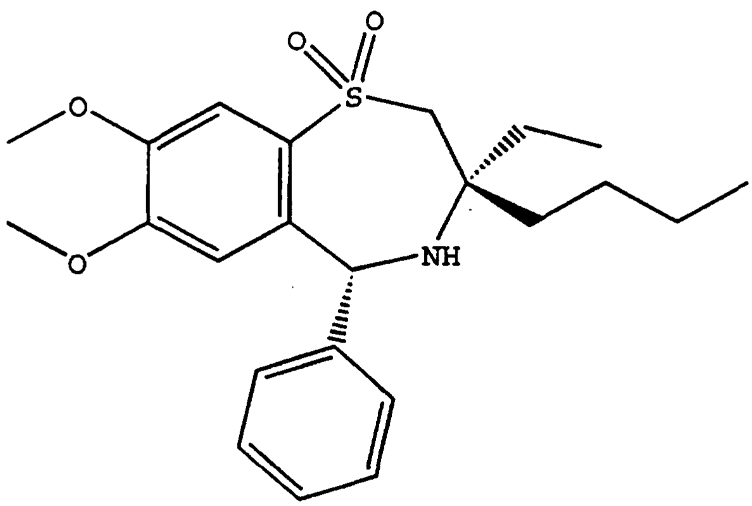

B-2

(3R,5R)-3-butyl-3-ethyl-2,3,4,5-tetrahydro-7,8-dimethoxy-5-phenyl-1-4-benzothiazepine 1,1-dioxide

Other benzothiazepine compounds useful for control of cholesterol are 2,3,4,5-tetrahydrobenzo-1-thi-5-azepine IBAT inhibitor compounds described in PCT Patent Application No. WO 99/35135. Included in that description is the compound of formula B-7.

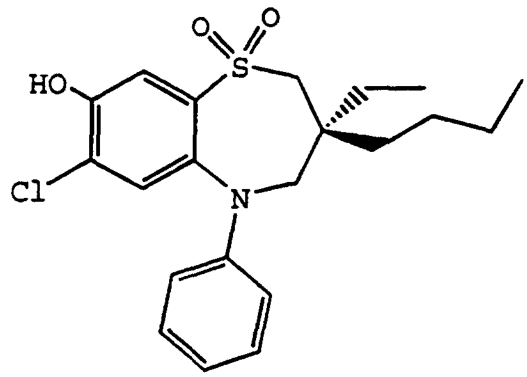

B-7

Further IBAT inhibitor compounds include a class of naphthalene IBAT inhibitor compounds, described by T. Ichihashi et al. in J. Pharmacol. Exp. Ther., 284(1), 43-50 (1998). In this class, S-8921 (methyl 1-(3,4-dimethoxyphenyl)-3-(3-ethylvaleryl)-4-hydroxy-6,7,8-trimethoxy-2-naphthoate) is particularly useful. The structure of S-8921 is shown in formula B-20. Further naphthalene compounds or lignin derivatives useful for the treatment or prophylaxis of hyperlipidemia or atherosclerosis are described in PCT Patent Application No. WO 94/24087.

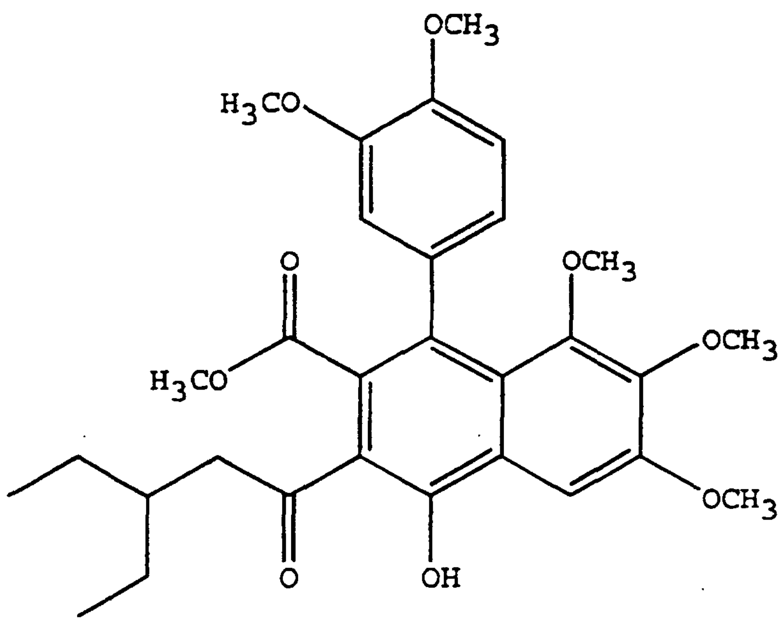

B-20

Another class of lipid-lowering drug is an antiobesity drug. An example of an antiobesity drug is orlistat. Orlistat is described in European Patent No. EP 0 129 748.

Inhibition of cholesteryl ester transfer protein (CETP) has been shown to effectively modify plasma HDL/LDL ratios, and is expected to check the progress and/or formation of certain cardiovascular diseases. CETP is a plasma protein that facilitates the movement of cholesteryl esters and triglycerides between the various lipoproteins in the blood (Tall, J. Lipid Res., 34, 1255-74 (1993)). The movement of cholesteryl ester from HDL to LDL by CETP has the effect of lowering HDL cholesterol. It therefore follows that inhibition of CETP should lead to elevation of plasma HDL cholesterol and lowering of plasma LDL cholesterol, thereby providing a therapeutically beneficial plasma lipid profile. Evidence of this effect is described in McCarthy, Medicinal Res. Revs., 13, 139-59 (1993). Further evidence of this effect is described in Sitori, *Pharmac. Ther.*, 67, 443-47 (1995)). This phenomenon was first demonstrated by Swenson et al., (J. Biol. Chem., 264, 14318 (1989)) with the use of a monoclonal antibody that specifically inhibits CETP. In rabbits, the antibody caused an elevation of the plasma HDL cholesterol and a decrease in LDL cholesterol. Son et al. (Biochim. Biophys. Acta, 795, 743-480 (1984)) describe proteins from human plasma that inhibit CETP. U.S. Patent 5,519,001, issued to Kushwaha et al., describes a 36 amino acid peptide derived from baboon apo C-1 that inhibits CETP activity. Cho et al. (*Biochim. Biophys. Acta* 1391, 133-144 (1998)) describe a peptide from hog plasma that

inhibits human CETP. Bonin et al. (*J. Peptide Res.*, 51, 216-225 (1998)) disclose a decapeptide inhibitor of CETP. A depspeptide fungal metabolite is disclosed as a CETP inhibitor by Hedge et al. in *Bioorg. Med. Chem. Lett.*, 8, 1277-80 (1998).

There have been several reports of non-peptidic compounds that act as CETP inhibitors. Barrett et al. (J. Am. Chem. Soc., 188, 7863-63 (1996)) describe cyclopropane-containing CETP inhibitors. Further cyclopropane-containing CETP inhibitors are described by Kuo et al. (J. Am. Chem. Soc., 117, 10629-34 (1995)). Pietzonka et al. (Bioorg. Med. Chem. Lett., 6, 1951-54 (1996)) describe phosphonate-containing analogs of cholesteryl ester as CETP inhibitors. Coval et al. (Bioorg. Med. Chem. Lett., 5, 605-610 (1995)) describe Wiedendiol-A and -B, and related sesquiterpene compounds as CETP inhibitors. Lee et al. (J. Antibiotics, 49, 693-96 (1996)) describe CETP inhibitors derived from an insect fungus. Busch et al. (Lipids, 25, 216-220, (1990)) describe cholesteryl acetyl bromide as a CETP inhibitor. Morton and Zilversmit (J. Lipid Res., 35, 836-47 (1982)) describe that p-chloromercuriphenyl sulfonate, p-hydroxymercuribenzoate and ethyl mercurithiosalicylate inhibit CETP. Connolly et al. (Biochem. Biophys. Res. Comm., 223, 42-47 (1996)) describe other cysteine modification reagents as CETP inhibitors. Xia et al. describe 1,3,5-triazines as CETP inhibitors (Bioorg. Med. Chem. Lett., 6, 919-22 (1996)). Bisgaier et al. (Lipids, 29, 811-8 (1994)) describe 4-phenyl-5-tridecyl-4H-1,2,4-triazole-thiol as a CETP inhibitor. Additional triazole CETP inhibitors are described in U.S. Patent Application Serial No. 09/153,360. Sikorski et al. disclosed further novel CETP inhibitors in PCT Patent Application No. WO 9914204.

Substituted 2-mercaptoaniline amide compounds can be used as CETP inhibitors and such therapeutic compounds are described by H. Shinkai et al. in PCT Patent Application No. WO 98/35937.

Some substituted heteroalkylamine compounds are known as CETP inhibitors. In European Patent Application No. 796846, Schmidt et al. describe 2-aryl-substituted pyridines as cholesterol ester transfer protein inhibitors useful as cardiovascular agents. One substituent at $C_3$ of the pyridine ring can be an hydroxyalkyl group. In European Patent Application No. 801060, Dow and Wright describe heterocyclic derivatives substituted with an aldehyde addition product of an alkylamine to afford 1-hydroxy-1-amines. These are reported to be β3-adrenergic receptor agonists useful for treating diabetes and other disorders. In Great Britain Patent Application No. 2305665, Fisher et al. disclose 3-agonist secondary amino alcohol substituted pyridine derivatives useful for treating several disorders including cholesterol levels and atherosclerotic diseases. In European Patent Application No. 818448 , Schmidt et al. describe tetrahydroquinoline derivatives as cholesterol ester transfer protein inhibitors. European Patent Application No. 818197, Schmek et al. describe pyridines with fused heterocycles as cholesterol ester transfer protein inhibitors. Brandes et al. in German Patent Application No. 19627430 describe bicyclic condensed pyridine derivatives as cholesterol ester transfer protein inhibitors. In PCT Patent Application No. WO 9839299, Muller-Gliemann et al. describe quinoline derivatives as cholesteryl ester transfer protein inhibitors.

Polycyclic compounds that are useful as CETP inhibitors are also disclosed by A. Oomura et al. in Japanese Patent No. 10287662. For example, therapeutic compounds having the structures C-1 and C-8 were prepared by culturing *Penicillium spp*.

Cycloalkylpyridines useful as CETP inhibitors are disclosed by Schmidt et al. in European Patent No. EP 818448. For example, the therapeutic compound having the structure C-9 is disclosed as being particularly effective as a CETP inhibitor.

Substituted tetrahydronaphthalene compounds useful as CETP inhibitors are described in PCT Patent Application No. WO 9914174. Specifically described in that disclosure as a useful CETP inhibitor is (8S)-3-cyclopentyl-1-(4-fluorophenyl)-2-[(S)-fluoro(4-trifluoromethylphenyl)methyl]-8-hydroxy-6-spirocclobutyl-5,6,7,8-tetrahydronaphthalene.

Some 4-heteroaryl-tetrahydroquinolines useful as CETP inhibitors are described in PCT Patent Application No. WO 9914215. For example, that disclosure describes 3-(4-trifluoromethylbenzoyl)-5,6,7,8-tetrahydroquinolin-5-one as a useful CETP inhibitor.

In another approach to the reduction of total cholesterol, use is made of the understanding that HMG CoA reductase catalyzes the rate-limiting step in the biosynthesis of cholesterol (The Pharmacological Basis of Therapeutics, 9th ed., J.G. Hardman and L.E. Limberd, ed., McGraw-Hill, Inc., New York, pp. 884-888 (1996).). HMG CoA reductase inhibitors (including the class of therapeutics commonly called "statins") reduce blood serum levels of LDL cholesterol by competitive inhibition of this biosynthetic step (M.S. Brown, et al., J. Biol. Chem, 253, 1121-28 (1978)). Several statins have been developed or commercialized throughout the world. Mevastatin was among the first of the statins to be developed and it is described in U.S. Patent No. 3,983,140 . Lovastatin, another important HMG CoA reductase inhibitor, is described in U.S. patent no. 4,231,938 . Simvastatin is described in U.S. patent no. 4,444,784. Each of these HMG CoA reductase inhibitors contains a six-membered lactone function which apparently mimics the structure of HMG CoA in competition for the reductase. The HMG CoA reductase inhibitor class of cholesterol-lowering drugs is further exemplified by a group of drugs which contain 2,4-dihydroxyheptanoic acid functionalities rather than the lactone. One member of this group is pravastatin, described in U.S. patent no. 4,346,227 . Another HMG CoA reductase inhibitor which contains a 2,4-dihydroxyheptanoic acid group is fluvastatin, described in U.S. patent no.

5,354,772 . Warnings of side effects from use of HMG CoA reductase inhibitors include liver dysfunction, skeletal muscle myopathy, rhabdomyolysis, and acute renal failure. Some of these effects are exacerbated when HMG CoA reductase inhibitors are combined with fibrates or nicotinic acid.

Fibric acid derivatives comprise another class of drugs which have effects on lipoprotein levels. Among the first of these to be developed was clofibrate, disclosed in U.S. patent no. 3,262,850. Clofibrate is the ethyl ester of p-chlorophenoxyisobutyric acid. A widely used drug in this class is gemfibrozil, disclosed in U.S. patent no. 3,674,836. Gemfibrozil frequently is used to decrease triglyceride levels or increase HDL cholesterol concentrations (The Pharmacological Basis of Therapeutics, p. 893). Fenofibrate (U.S. patent no. 4,058,552) has an effect similar to that of gemfibrozil, but additionally decreases LDL levels. Ciprofibrate (U.S. patent no. 3,948,973) has similar effects to that of fenofibrate. Another drug in this class is bezafibrate (U.S. patent no. 3,781,328). Warnings of side effects from use of fibric acid derivatives include gall bladder disease (cholelithiasis), rhabdomyolysis, and acute renal failure. Some of these effects are exacerbated when fibrates are combined with HMG CoA reductase inhibitors.

Probucol is a powerful antioxidant which has shown the ability to lower serum cholesterol levels and cause regression of xanthomas in patients having homozygous familial hypercholesterolemia (A. Yamamoto, et al., Am. J. Cardiol., 57, 29H-35H (1986)). However, treatment with probucol alone sometimes shows erratic control of LDL and frequent lowering of HDL (The Pharmacological Basis of Therapeutics, p. 891). Probucol is contraindicated for patients with progressive myocardial damage and/or ventricular arrhythmias.

A class of materials which operates by another mechanism to lower LDL cholesterol comprises bile acid sequestering agents. Such agents are typically anion exchange polymers administered orally to a patient. As the agent passes through the gut, anions of bile acids are sequestered by the agent and excreted. Such sequestering has been speculated to prevent reabsorption by the gut, for example the ileum, thereby preventing conversion of the bile acids into cholesterol. One such bile acid sequestering agent is cholestyramine, a styrenedivinylbenzene copolymer containing quaternary ammonium cationic groups capable of binding bile acids. It is believed that cholestyramine binds the bile acids in the intestinal tract, thereby interfering with their normal enterohepatic circulation. This effect is described by Reihnér et al., in "Regulation of hepatic cholesterol metabolism in humans: stimulatory effects of cholestyramine on HMG-CoA reductase activity and low density lipoprotein receptor expression in gallstone patients", Journal of Lipid Research, 31, 2219-2226 (1990). Further description of this effect is found in Suckling et al. in "Cholesterol Lowering and bile acid excretion in the hamster with cholestyramine treatment", Atherosclerosis, 89, 183-90 (1991). This results in an increase in liver bile acid synthesis because of the liver using cholesterol as well as an upregulation of the liver LDL receptors which enhances clearance of cholesterol and decreases serum LDL cholesterol levels.

Another bile acid sequestering agent is colestipol, a copolymer of diethylenetriamine and 1-chloro-2,3-epoxypropane. Colestipol is described in U.S. Patent No. 3,692,895. A frequent side effect of colestipol and of cholestyramine is gastric distress.

Additional bile acid sequestering agents are described in U.S. Patent No. 5,703,188, assigned to Geltex Pharmaceuticals Inc. For example, one such bile acid sequestering agent is 3-methacrylamidopropyltrimethylammonium chloride copolymerized with ethylene glycol dimethacrylate to yield a copolymer.

Yet another class materials proposed as bile acid sequestering agents comprises particles comprising amphiphilic copolymers having a crosslinked shell domain and an interior core domain (Patent application no. PCT/US 97/11610). Structures and preparation of such crosslinked amphiphilic copolymers are described in PCT/US97/11345. Such particles have been given the common name of "knedels" (K.B. Thurmond et al., J. Am. Chem. Soc., 118 (30), 7239-40 (1996)).

Nicotinic acid (niacin) is a B-complex vitamin reported as early as 1955 to act as a hypolipidemic agent (R. Altschl, et al., Arch. Biochem. Biophys., 54, 558-9 (1955)). It is sometimes used to raise low HDL levels and lower VLDL and LDL levels. Useful commercial formulations of nicotinic acid include Niacor, Niaspan, Nicobid, Nicolar, Slo-Niacin. Nicotinic acid is contraindicated for patients having hepatic dysfunction, active peptic ulcer, or arterial bleeding. Another compound in this class useful for cardiovascular indications is niceritrol (T. Kazumi et al., Curr. Ther. Res., 55, 546-51). J. Sasaki et al. (Int. J. Clin. Pharm. Ther., 33 (7), 420-26 (1995)) describes a reduction in cholesterol ester transfer activity by niceritrol monotherapy. Acipimox (5-methyl pyrazine-2-carboxylic acid 4-oxide, U.S. Patent No. 4,002,750) is structurally similar to nicotinic acid and has antihyperlipidemic activity.

A study by Wetterau et al. (Science, 282, 751-54 (1998)) describes a number of alkylpiperidine compounds, isoindole compounds, and fluorene compounds useful for inhibiting microsomal triglyceride transfer protein (MTP inhibitors). Rodents and Watanabe-heritable hyperlipidemic rabbits treated with these compounds show decreased production of lipoprotein particles.

Cholesterol absorption antagonists may also be useful for the treatment of prophylaxis of cardiovascular diseases such as hypercholesterolemia or atherosclerosis. For example, azetidinones such as SCH 58235 ([3R-[3α(S*), 4β]]-1-(4-fluorophenyl)-3-[3-(4-fluorophenyl)-3-hydroxypropyl]-4-(4-hydroxyphenyl)-2-azetidinone) (formula A-1), described in J. Med. Chem., 41(6), 973-980 (1998), are useful cholesterol absorption antagonists. SCH 58235 is further described by Van Heek et al. in J. Pharmacol. Exp. Ther., 283(1), 157-163 (1997). Further azetidinone compounds

useful for treatment or prophylaxis of cardiovascular disease are described in U.S. Patent No. 5,767,115.

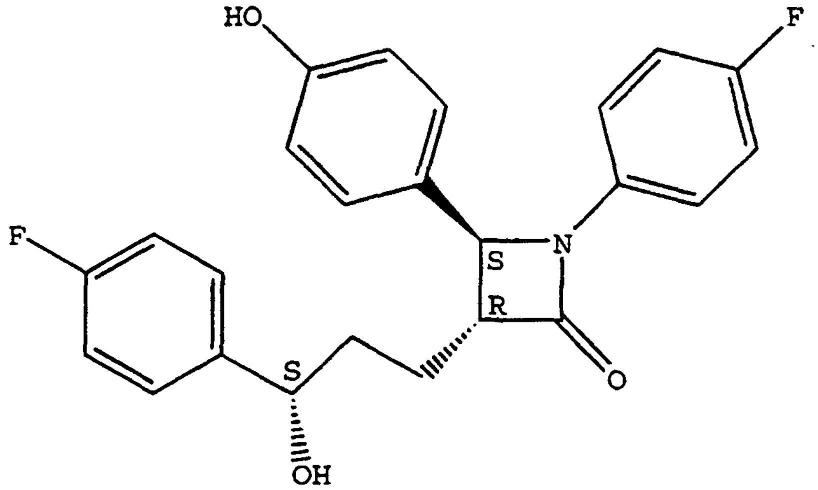

A-1

[3R-[3a(S*),4b]]-1-(4-fluorophenyl)-3-[3-(4-fluorophenyl)-3-hydroxypropyl]-4-(4-hydroxyphenyl)-2-azetidinone

Phytosterols, and especially stanols have been shown to effectively inhibit cholesterol absorption from the gastrointestinal tract, and to negatively affect cholesterol synthesis. Phytosterols are expected to slow or inhibit the progress and formation of certain cardiovascular conditions, including hyperlipidemic conditions such as hypercholesterolemia and atherosclerosis. Stanols are 5α saturated derivatives of phytosterols. (Straub, U.S. Patent No. 5,244,887). It has been suggested that phytosterols lower blood cholesterol levels by reducing the absorption of cholesterol from the intestine (Ling and Jones, "Minireview Dietary Phytosterols: A Review of Metabolism, Benefits and Side Effects," Life Sciences, 57 (3), 195-206 (1995)).

Sitostanol, clionastanol, 22,23-dihydrobrassicastanol, campestanol, and mixtures thereof contained in food additives intended to reduce cholesterol absorption from foods and beverages containing cholesterol are described by Straub in U.S. Patent Number 5,244,887.

A beta-sitostanol fatty acid ester or fatty acid ester mixture which lowers cholesterol in serum is described by Miettinen et al. in U.S. Patent Number 5,502,045.

A stanol composition containing in sitostanol and campestanol which effectively lowers serum cholesterol levels when incorporated into edibles is described by Wester et al. in WO 9806405.

A therapeutic composition of one or more oxysterols and a suitable carrier to inhibit cholesterol absorption from the diet is described by Haines in U.S. Patent Number 5,929,062.

Cardiovascular disease is also caused or aggravated by hypertension. Hypertension is defined as persistently high blood pressure. Generally, adults are classified as being hypertensive when systolic blood pressure is persistently above 140 mmHg or when diastolic blood pressure is above 90 mmHg. Long-term risks for cardiovascular mortality increase in a direct relationship with persistent blood pressure (E. Braunwald, Heart Disease, 5th ed., W.B. Saunders & Co., Philadelphia, 1997, pp. 807-823). Various mechanisms have been advantageously exploited to control hypertension. For example, useful antihypertensive agents can include, without limitation, an andrenergic blocker, a mixed alpha/beta andrenergic blocker, an alpha andrenergic blocker, a beta andrenergic blocker, an andrenergic stimulant, an angiotensin converting enzyme (ACE) inhibitor, an angiotensin II receptor antagonist, a calcium channel blocker, a diuretic, or a vasodilator. A particularly useful antihypertensive agent is eplerenone (see, for example, U.S. Patent No. 4,559,332). Eplerenone lowers blood pressure by functioning as a diuretic. Eplerenone was formerly called epoxymexrenone.

Some combination therapies for the treatment of cardiovascular disease have been described in the literature. Combinations of IBAT inhibitors with HMG CoA reductase inhibitors useful for the treatment of cardiovascular disease are disclosed in U.S. Patent Application No. 09/037,308 and in PCT Patent Application No. 98/40375.

A combination therapy of fluvastatin and niceritrol is described by J. Sasaki et al. (Id.). Those researchers conclude that the combination of fluvastatin with niceritrol "at a dose of 750 mg/day dose does not appear to augment

or attenuate beneficial effects of fluvastatin."

L. Cashin-Hemphill et al. (J. Am. Med. Assoc., 264 (23), 3013-17 (1990)) describe beneficial effects of a combination therapy of colestipol and niacin on coronary atherosclerosis. The described effects include nonprogression and regression in native coronary artery lesions.

A combination therapy of acipimox and simvastatin shows beneficial HDL effects in patients having high triglyceride levels (N. Hoogerbrugge et al., J. Internal Med., 241, 151-55 (1997)).

Sitostanol ester margarine and pravastatin combination therapy is described by H. Gylling et al. (J. Lipid Res., 37, 1776-85 (1996)). That therapy is reported to simultaneously inhibit cholesterol absorption and lower LDL cholesterol significantly in non-insulin-dependent diabetic men.

Brown et al. (New Eng. J. Med., 323 (19), 1289-1339 (1990)) describe a combination therapy of lovastatin and colestipol which reduces atherosclerotic lesion progression and increase lesion regression relative to lovastatin alone.

Scott (PCT Patent Application No. WO 99/11260) describes combinations of atorvastatin (an HMG CoA reductase inhibitor) with an antihypertensive agent for the treatment of angina pectoris, atherosclerosis, combined hypertension and hyperlipidemia, and symptoms of cardiac risk.

Egan et al. (PCT Patent Application No. WO 96/40255) describe a combination therapy of an angiotension II antagonist and an epoxy-steroidal aldosterone antagonist. The epoxy-steroidal aldosterone antagonist in the Egan application includes eplerenone.

The above references show continuing need to find safe, effective agents for the prophylaxis or treatment of cardiovascular diseases.

## Summary of the Invention

To address the continuing need to find safe and effective agents for the prophylaxis and treatment of cardiovascular diseases, combination therapies of cardiovascular drugs are now reported.

The present invention provides a therapeutic composition comprising first amount of an IBAT inhibitor and a second amount of a microsomal triglyceride transfer protein inhibitor (MTP inhibitor), wherein the first and second amounts together comprise an anti-hyperlipidemic condition effective amount or an anti-atherosclerotic condition effective amount of the compounds. The IBAT inhibitor in the embodiments of this invention is preferably a benzothiepine IBAT inhibitor. In another embodiment, the IBAT inhibitor can be a benzothiazepine IBAT inhibitor. In still another embodiment, the IBAT inhibitor can be a naphthalene IBAT inhibitor.

Among its several embodiments, the present invention further provides a combination comprising a first amount of an IBAT inhibitor and a second amount of another cardiovascular therapeutic useful in the prophylaxis or treatment of hyperlipidemia or atherosclerosis, wherein the first and second amounts together comprise an anti-hyperlipidemic condition effective amount or an anti-atherosclerotic condition effective amount of the compounds.

A still further embodiment of the instant invention comprises the use of any of the cardiovascular combination therapies described herein in the manufacture of a medicament for the prophylaxis or treatment of hypercholesterolemia or atherosclerosis.

In another embodiment the present invention provides the use, for the manufacture of a medicament for the prophylaxis or treatment of a hyperlipidemic condition or disorder in a mammal, of a first amount of an ileal bile acid transport inhibitor compound and a second amount of a microsomal triglyceride transfer protein inhibiting compound wherein the first amount and the second amount together comprise an anti-hyperlipidemic condition effective amount, an anti-atherosclerotic condition effective amount, or an anti-hypercholesterolemic condition effective amount of the compounds.

In another embodiment the present invention provides a kit for achieving a therapeutic effect in a mammal comprising an amount of an ileal bile acid transport inhibiting compound in a first unit dosage form; an amount of a microsomal triglyceride transfer protein inhibiting compound in a second unit dosage form; and container means for containing said first and second unit dosage forms.

Further scope of the applicability of the present invention will become apparent from the detailed description provided below. However, it should be understood that the following detailed description and examples, while indicating preferred embodiments of the invention, are given by way of illustration only since various changes and modifications within the scope of the invention will become apparent to those skilled in the art from this detailed description.

## DETAILED DESCRIPTION OF THE PREFERRED EMBODIMENTS

The following detailed description is provided to aid those skilled in the art in practicing the present invention. Even so, this detailed description should not be construed to unduly limit the present invention as modifications and variations in the embodiments discussed herein can be made by those of ordinary skill in the art without departing from

the scope of the present inventive discovery.

## a. Definitions

The following definitions are provided in order to aid the reader in understanding the detailed description of the present invention:

"Benzothiepine IBAT inhibitor" means an ileal bile acid transport inhibitor which comprises a therapeutic compound comprising a 2,3,4,5-tetrahydro-1-benzothiepine 1,1-dioxide structure or a 2,3,4,5-tetrahydro-1-benzothiepine 1-oxide structure.

"Benzothiazepine IBAT inhibitor" means an ileal bile acid transport inhibitor which comprises a therapeutic compound comprising a 2,3,4,5-tetrahydro-1-benzothi-4-azepine 1,1-dioxide structure or a 2,3,4,5-tetrahydro-1-benzothi-5-azepine 1,1-dioxide structure.

"Naphthalene IBAT inhibitor" means an ileal bile acid transport inhibitor which comprises a therapeutic compound comprising a substituted naphthalene structure.

"Combination therapy" means the administration of two or more therapeutic agents to treat a hypertensive condition or a hyperlipidemic condition, for example atherosclerosis and hypercholesterolemia. Such administration encompasses co-administration of these therapeutic agents in a substantially simultaneous manner, such as in a single dosage form having a fixed ratio of active ingredients or in multiple, separate dosage forms for each inhibitor agent. In addition, such administration also encompasses use of each type of therapeutic agent in a sequential manner. In either case, the treatment regimen will provide beneficial effects of the drug combination in treating the hypertensive condition or the hyperlipidemic condition.

The phrase "therapeutically effective" is intended to qualify the combined amount of inhibitors in the combination therapy. This combined amount will achieve the goal of reducing or eliminating the hypertensive condition or the hyperlipidemic condition.

"Therapeutic compound" means a compound useful in the prophylaxis or treatment of a hypertensive condition or a hyperlipidemic condition, including atherosclerosis and hypercholesterolemia.

## b. Combinations

The combinations of the present invention will have a number of uses. For example, through dosage adjustment and medical monitoring, the individual dosages of the therapeutic compounds used in the combinations of the present invention will be lower than are typical for dosages of the therapeutic compounds when used in monotherapy. The dosage lowering will provide advantages including reduction of side effects of the individual therapeutic compounds when compared to the monotherapy. In addition, fewer side effects of the combination therapy compared with the monotherapies will lead to greater patient compliance with therapy regimens.

Another use of the present invention will be in combinations having complementary effects or complementary modes of action. For example, IBAT inhibitors frequently lower LDL lipoprotein but also lower HDL lipoprotein. In contrast, CETP (cholesteryl ester transfer protein) inhibitors raise HDL. A therapeutic combination of an IBAT inhibitor and a CETP inhibitor will, when dosages are optimally adjusted, lower LDL yet maintain or raise HDL.

Compounds useful in the present invention encompass a wide range of therapeutic compounds. IBAT inhibitors useful in the present invention are disclosed in patent application no. PCT/US95/10863. More IBAT inhibitors are described in PCT/US97/04076. Still further IBAT inhibitors useful in the present invention are described in U.S. Application Serial No. 08/816,065. More IBAT inhibitor compounds useful in the present invention are described in WO 98/40375. Additional IBAT inhibitor compounds useful in the present invention are described in U.S. Application Serial No. 08/816,065. IBAT inhibitors of particular interest in the present invention are shown in Table 1, as well as the diastereomers, enantiomers, racemates, salts, and tautomers of the IBAT inhibitors of Table 1.

Table 1.

| Compound Number | Structure |
|---|---|
| B-1 | 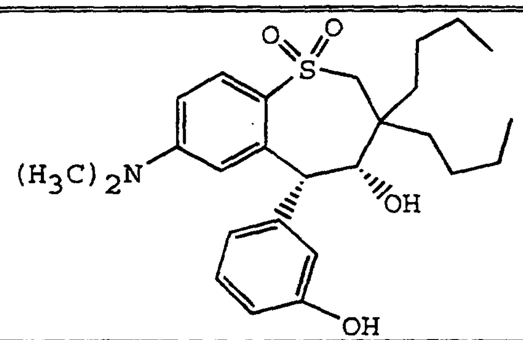 |
| B-2 | 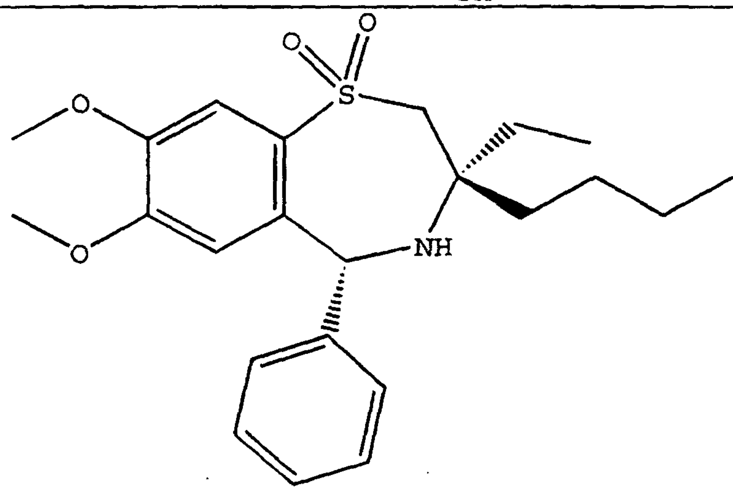 (3R,5R)-3-butyl-3-ethyl-2,3,4,5-tetrahydro-7,8-dimethoxy-5-phenyl-1-4-benzothiazepine 1,1-dioxide |
| | |

| | |
|---|---|
| B-3 | 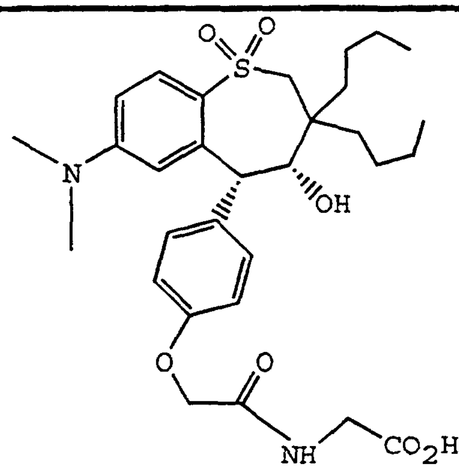 |
| B-4 | 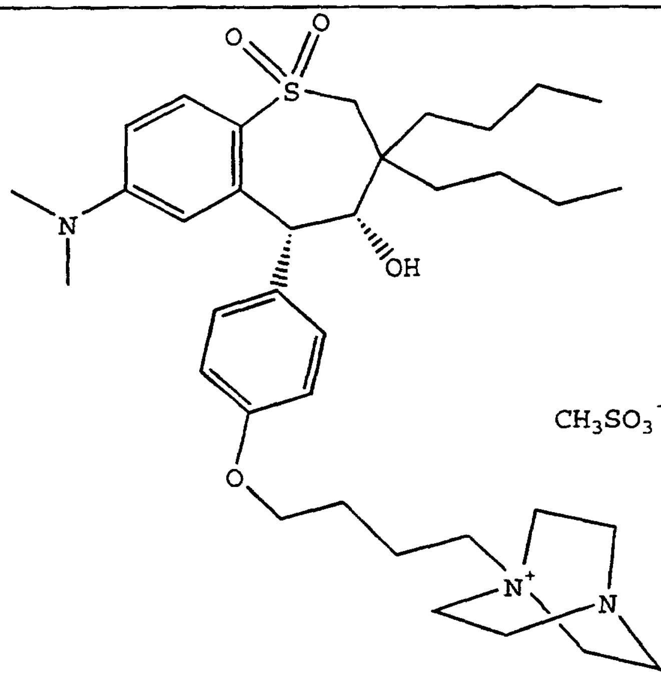 $CH_3SO_3^-$ |
| B-5 | 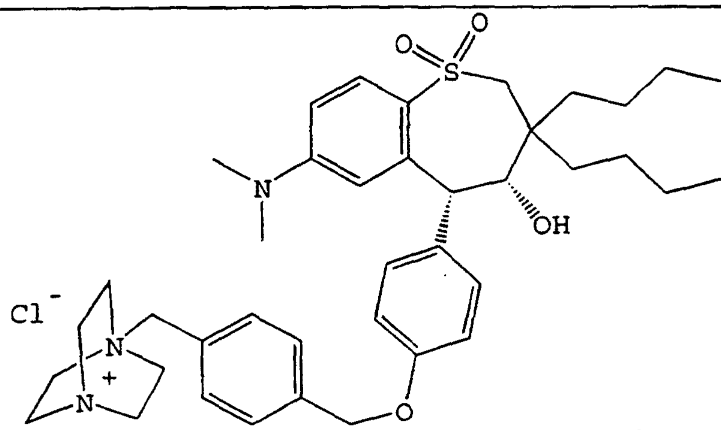 $Cl^-$ |

| | |
|---|---|
| B-6 | 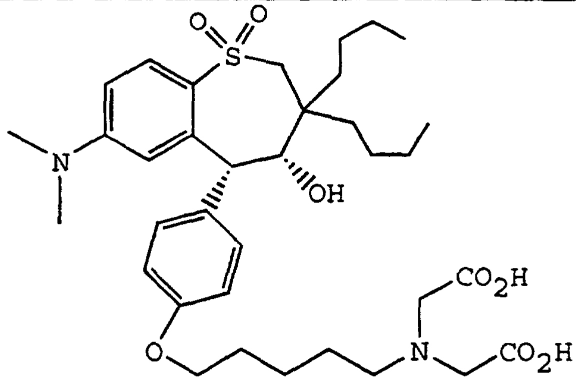 |
| B-7 | 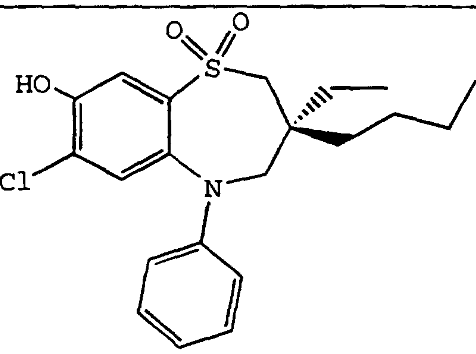 |
| B-8 | 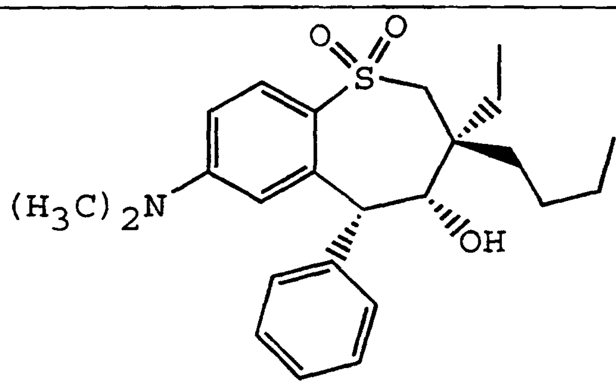 |
| B-9 | 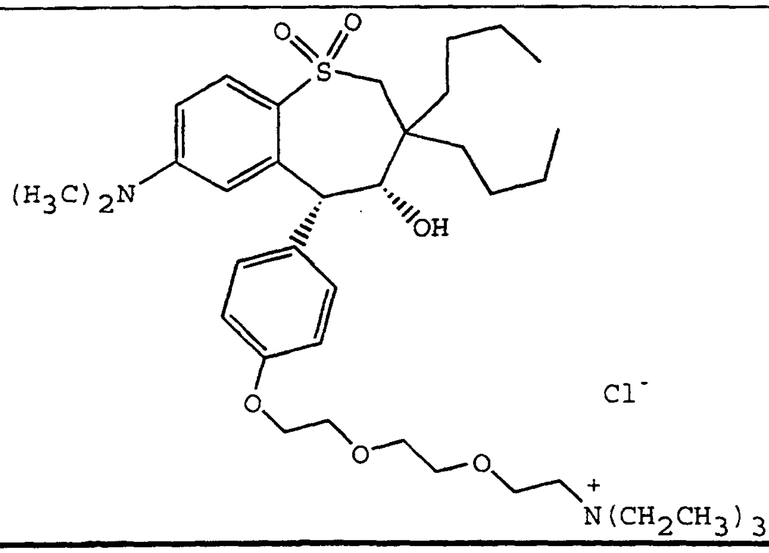 |

| | |
|---|---|
| B-10 | 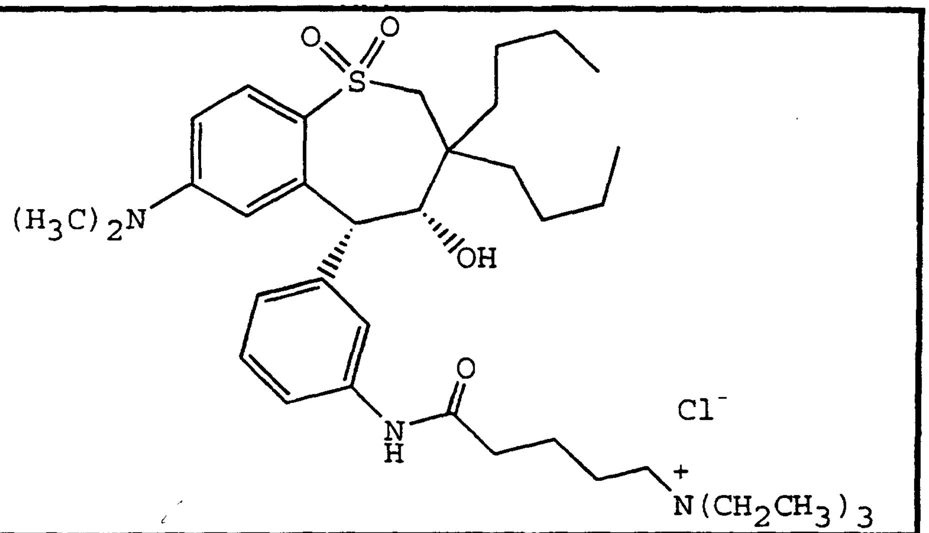 |
| B-11 | 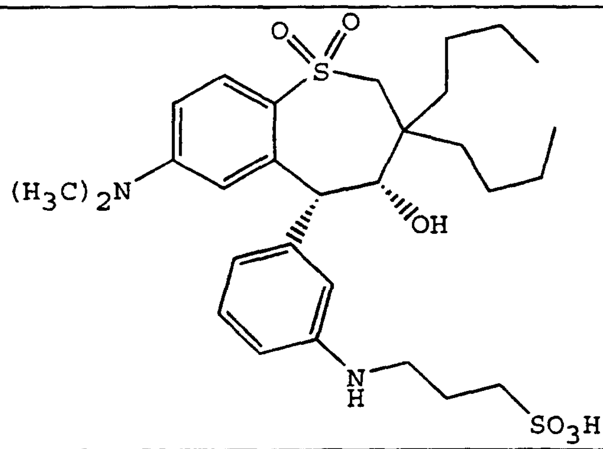 |
| B-12 | 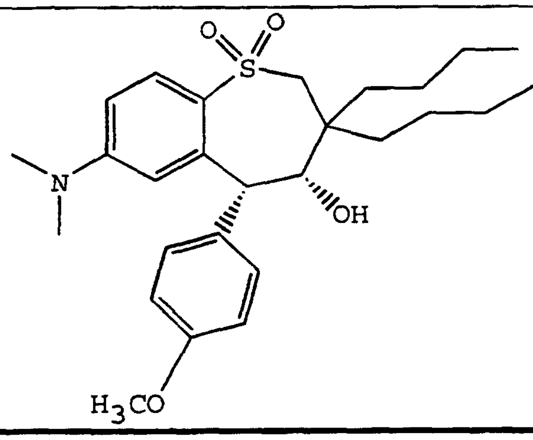 |

| | |
|---|---|
| B-13 | 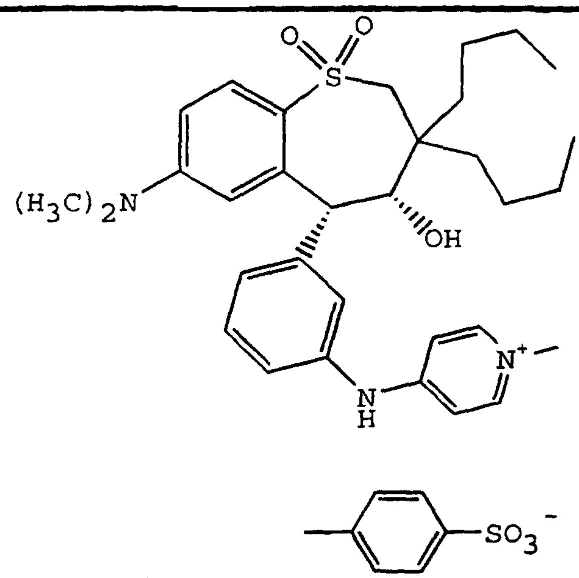 |
| B-14 | 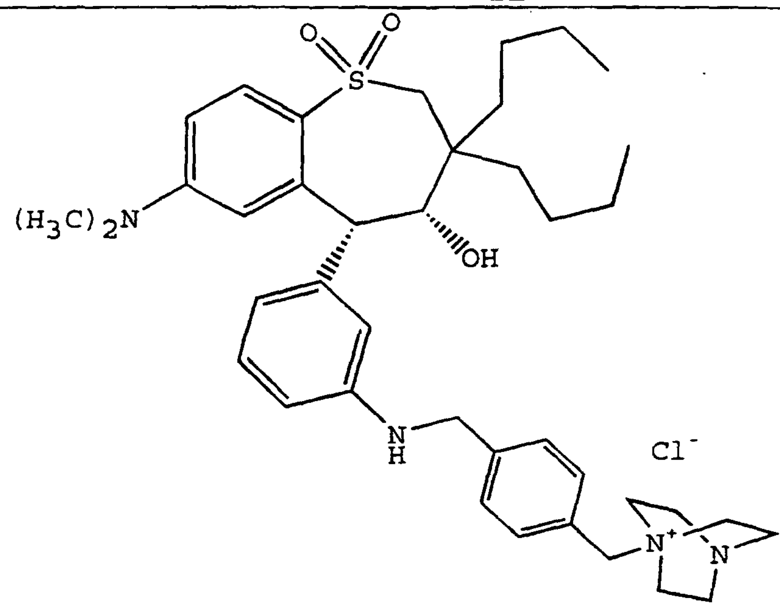 |

| | |
|---|---|
| B-15 | 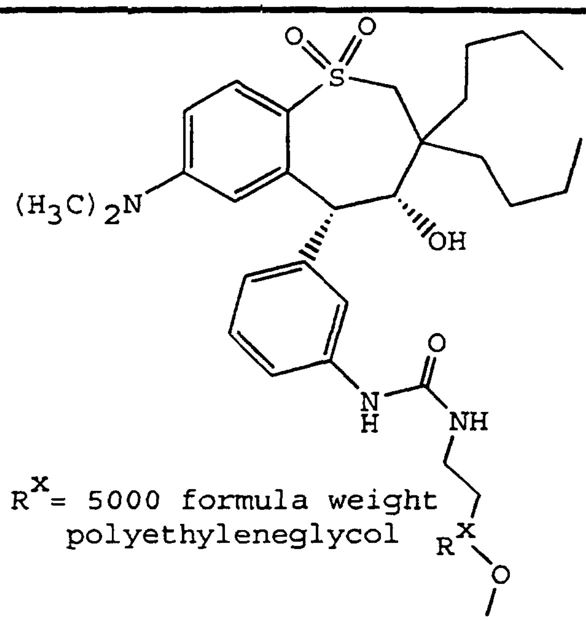<br>$R^x$ = 5000 formula weight polyethyleneglycol |
| B-16 | 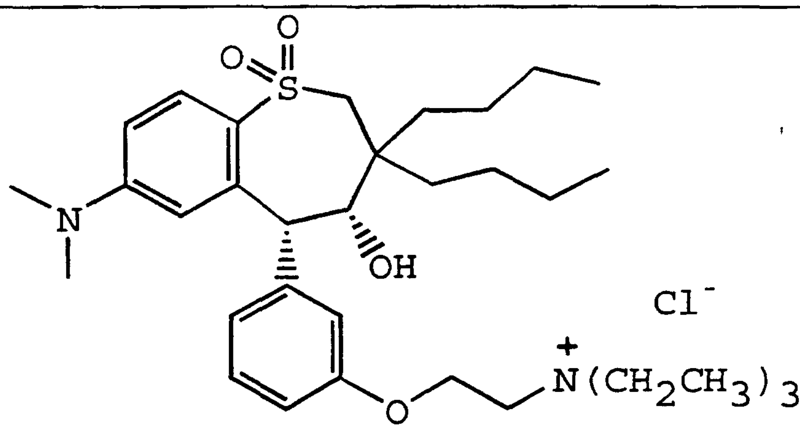 |
| B-17 | 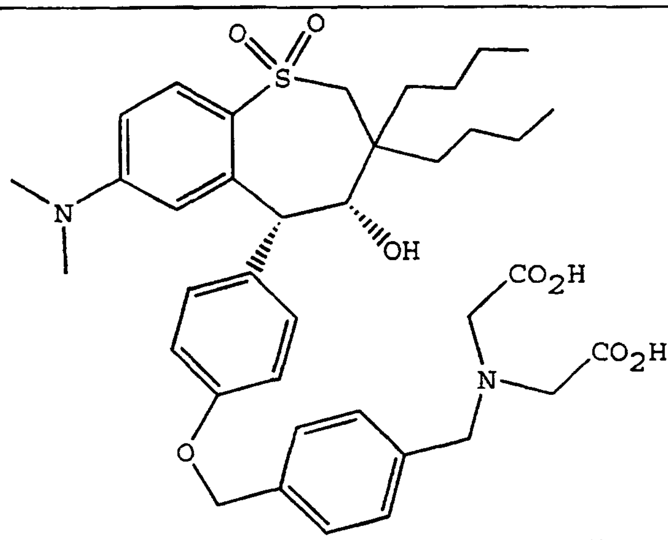 |

| | |
|---|---|
| B-18 | 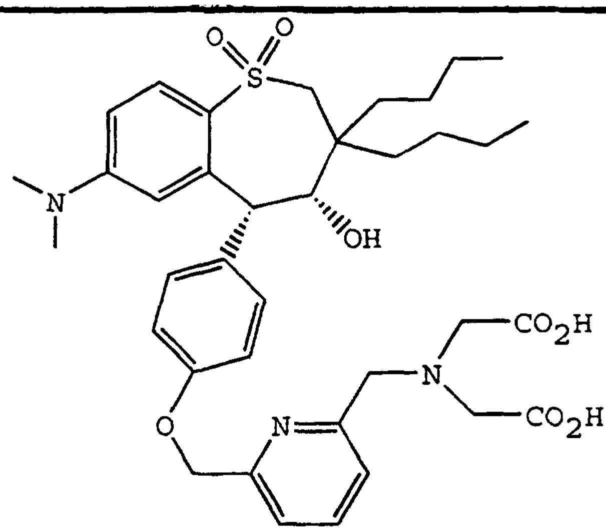 |
| B-19 | 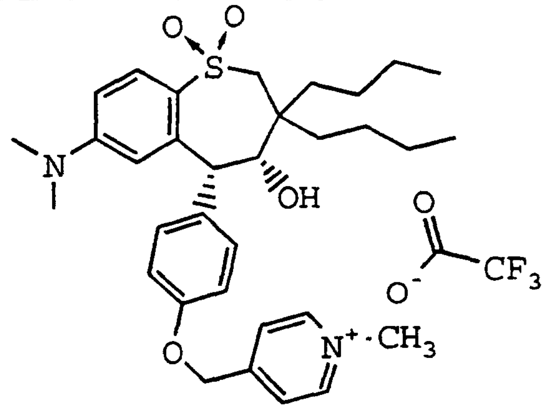 |
| B-20 | 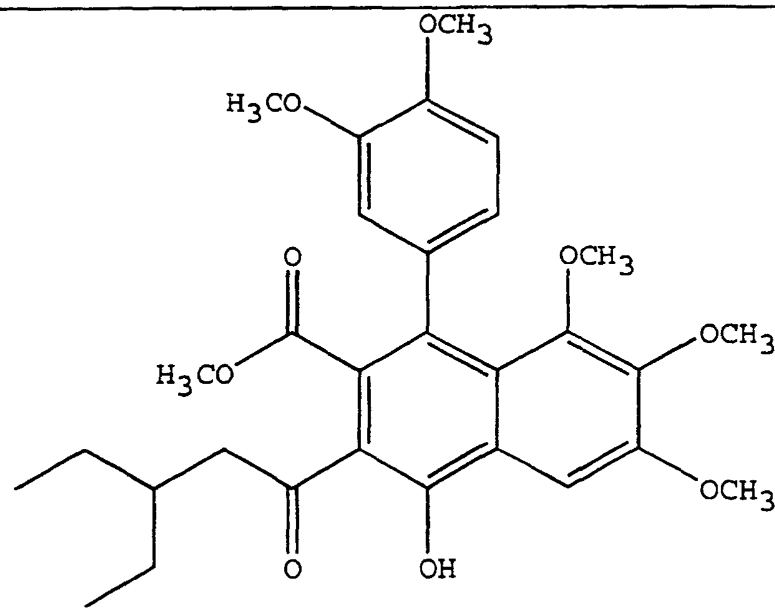 |

| | |
|---|---|
| B-21 | 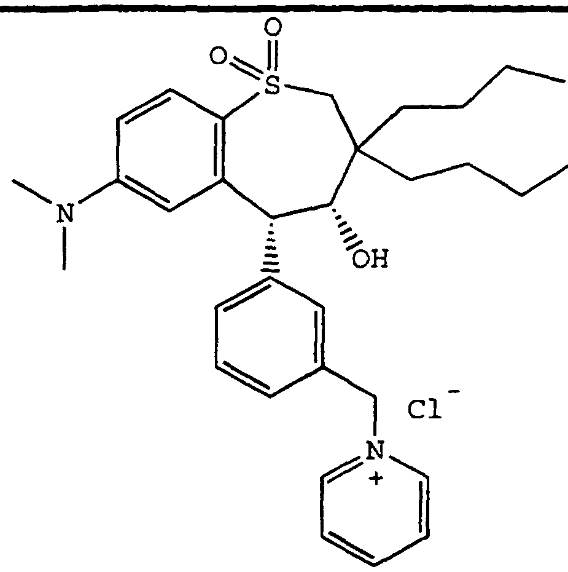 |
| B-22 | 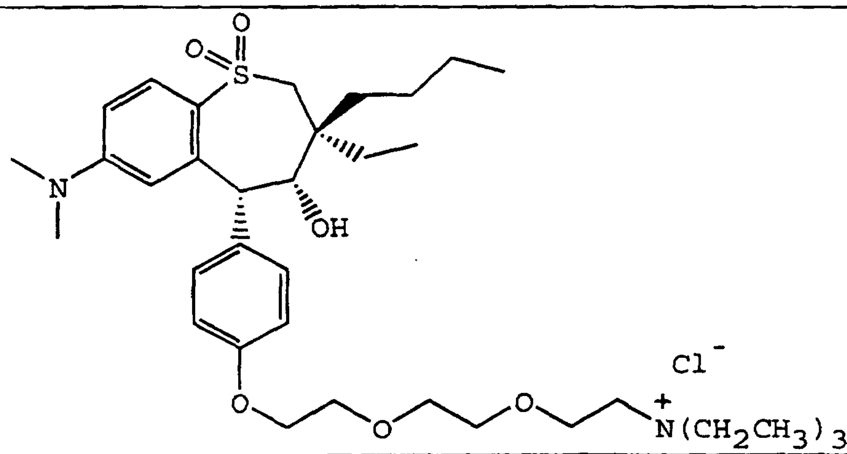 |
| B-23 | 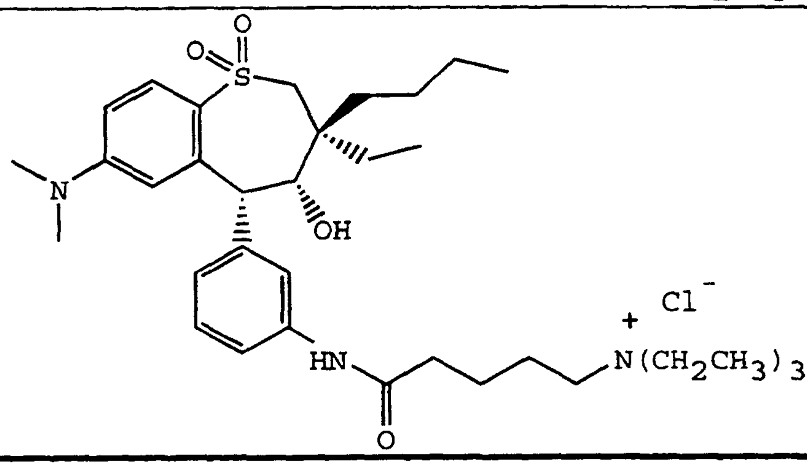 |

| | |
|---|---|
| B-24 | 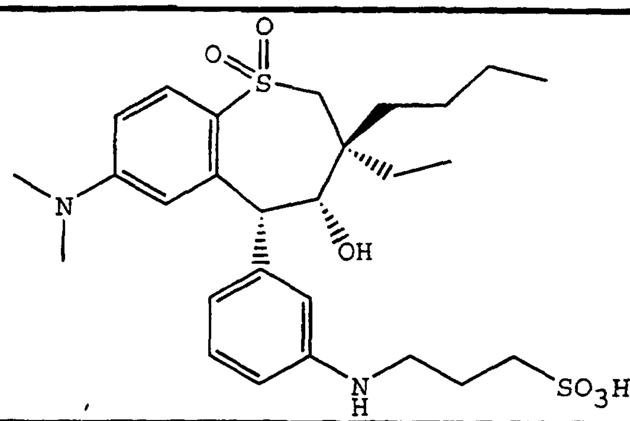 |
| B-25 | 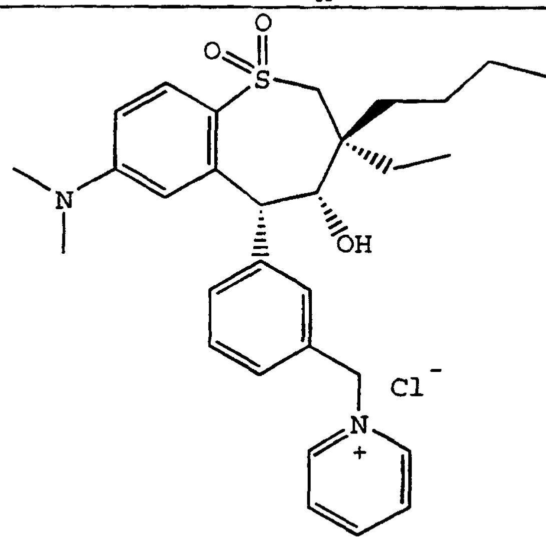 |
| B-26 | 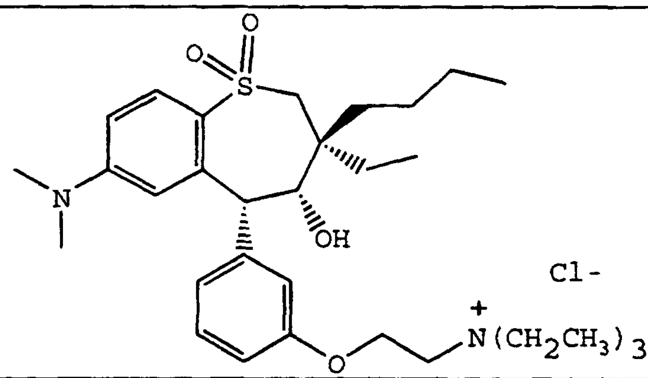 |
| B-27 | 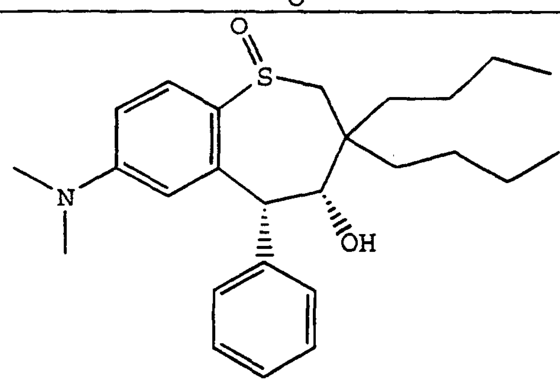 |

| | |
|---|---|
| B-28 | 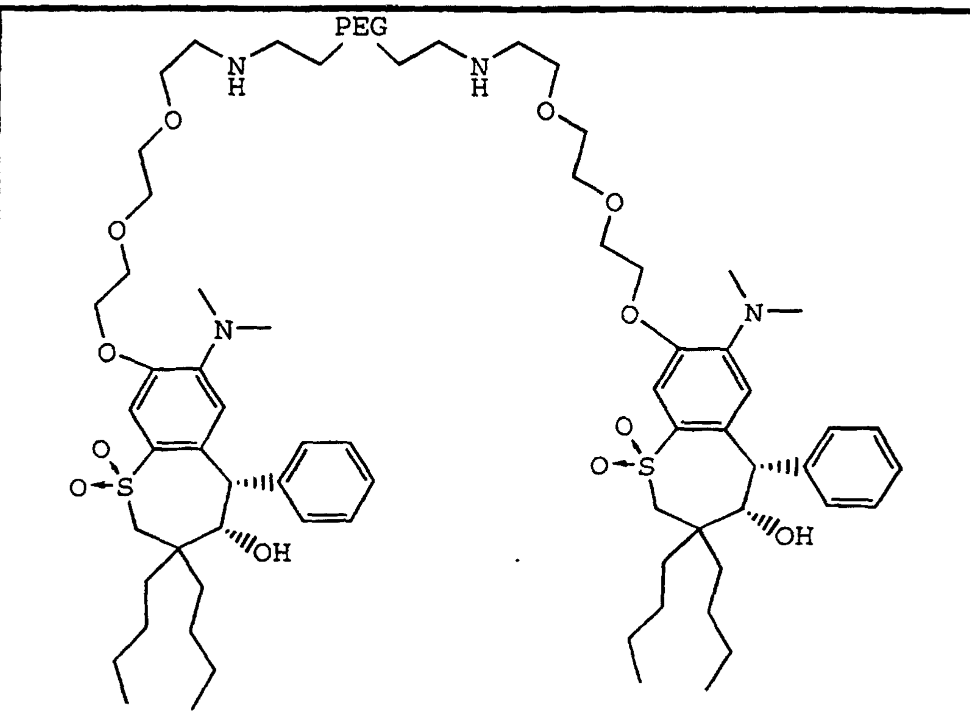 PEG = 3400 molecular weight polyethylene glycol polymer chain |
| B-29 | 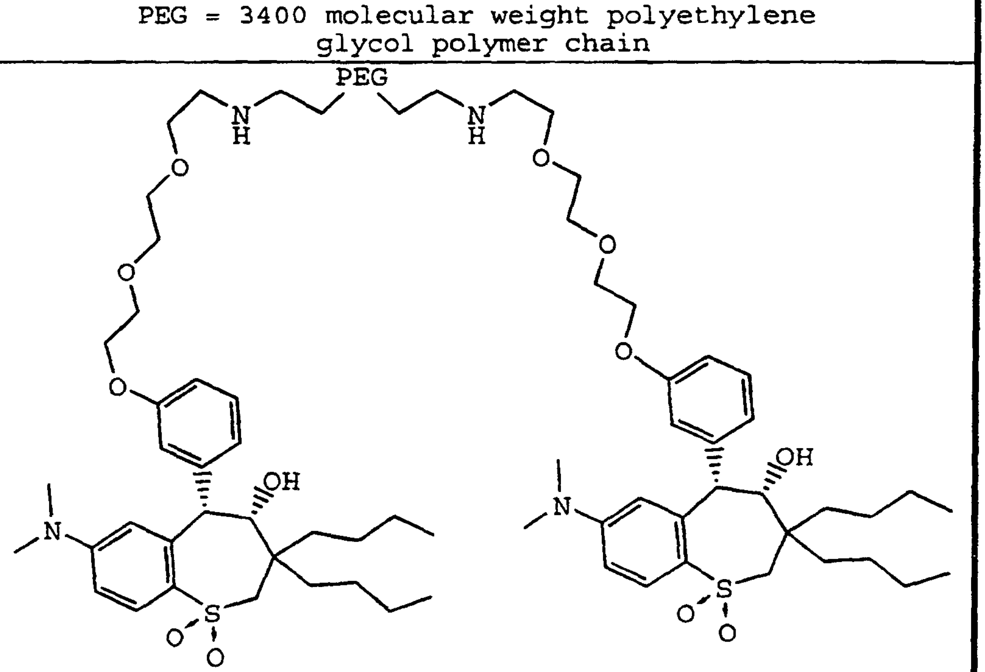 PEG = 3400 molecular weight polyethylene glycol polymer chain |

| | |
|---|---|
| B-30 | 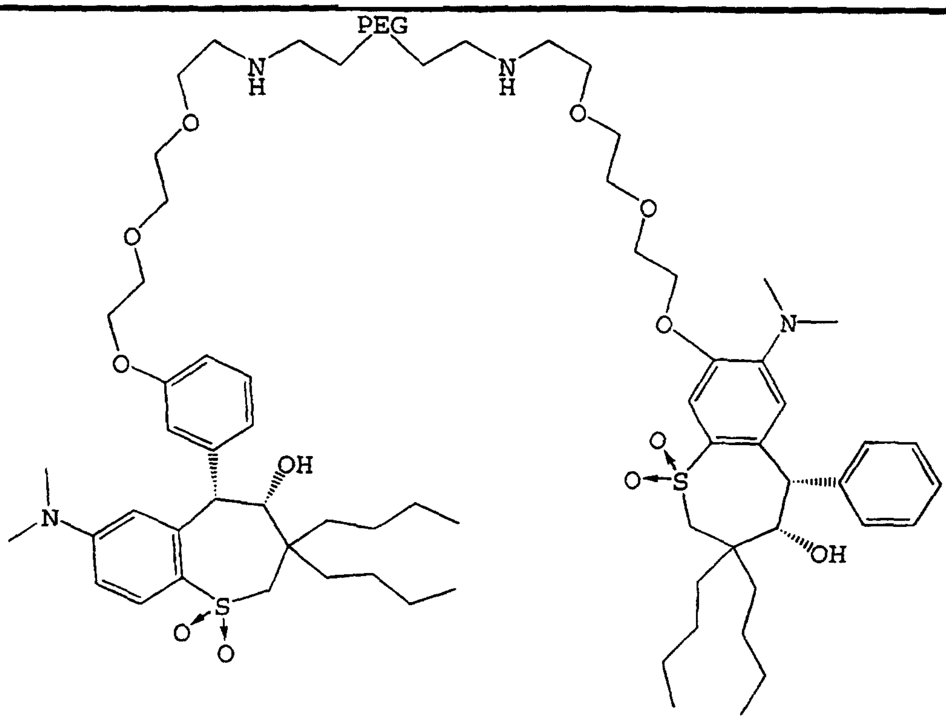<br>PEG = 3400 molecular weight polyethylene glycol polymer chain |
| B-31 | 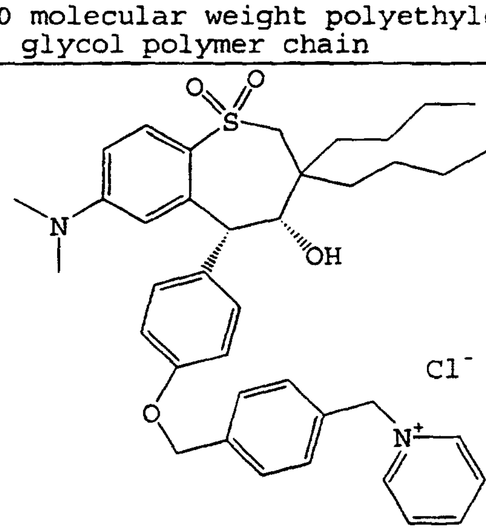 |
| B-32 | 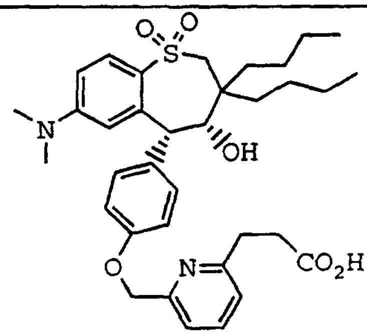 |

| | |
|---|---|
| B-33 | 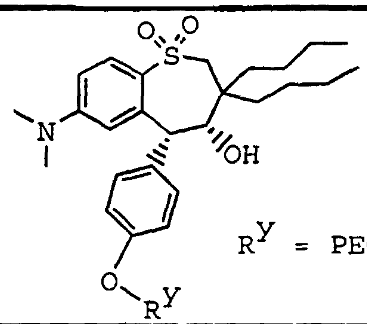 $R^Y$ = PEG 1000 |
| B-34 | 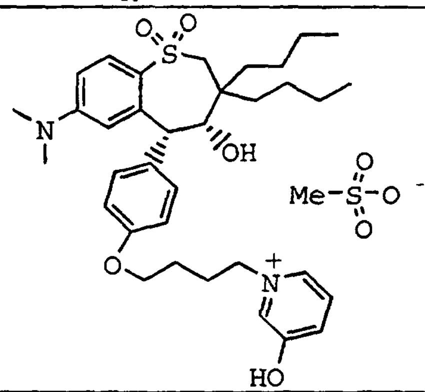 |
| B-35 | 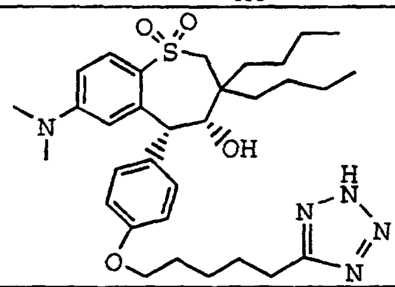 |
| B-36 | 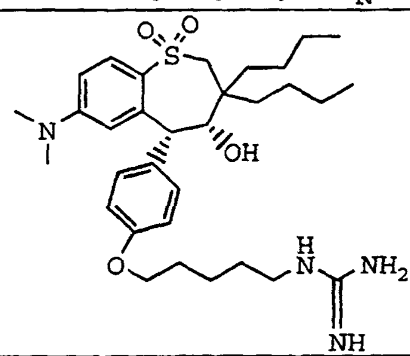 |
| B-37 | 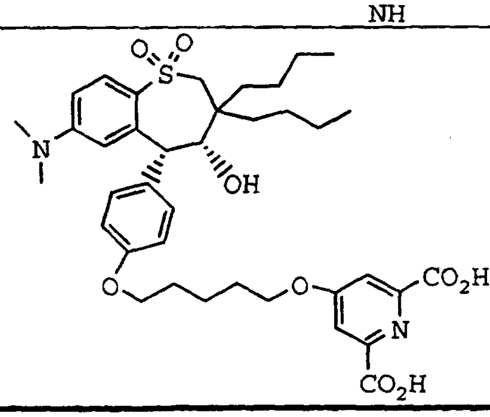 |

| | |
|---|---|
| B-38 | 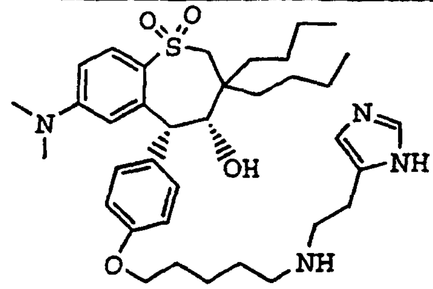 |
| B-39 | 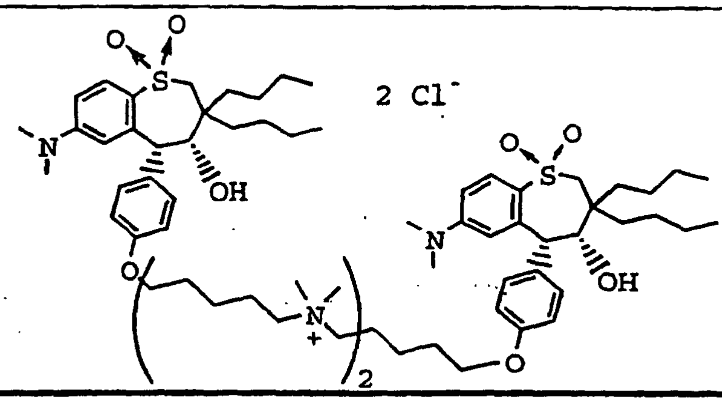 |

MTP inhibitor compounds useful in the combinations and methods of the present invention comprise a wide variety of structures and functionalities. Some of the MTP inhibitor compounds of particular interest for use in the present invention are shown in Table 4b. The therapeutic compounds of Table 4b can be used in the present invention in a variety of forms, including acid form, salt form, racemates, enantiomers, zwitterions, and tautomers. Descriptions of the therapeutic compounds of Table 4b can be found in Science, 282, 23 October 1998, pp. 751-754.

Table 4b.

| Compound Number | Structure |
|---|---|
| M-1 | 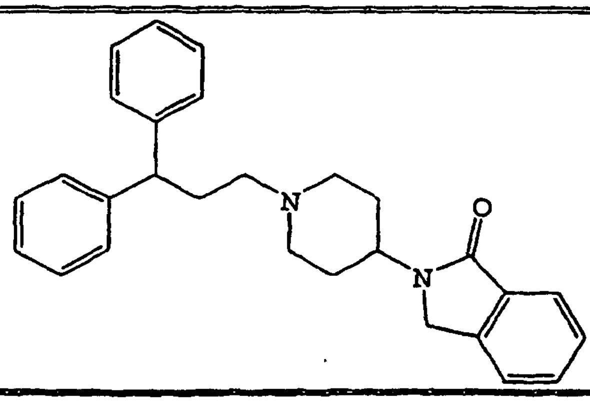 |

| | |
|---|---|
| M-2 | 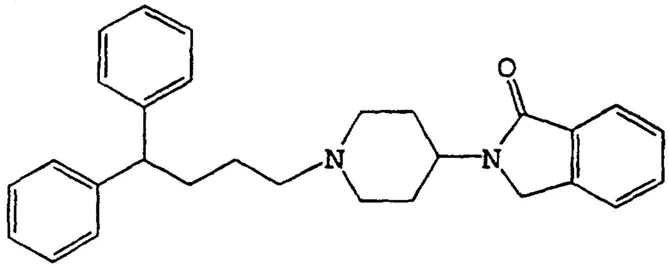 |
| M-3 | 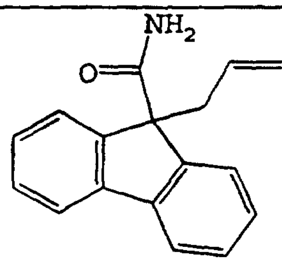 |
| M-4 | 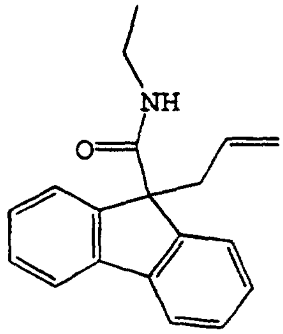 |
| M-5 | 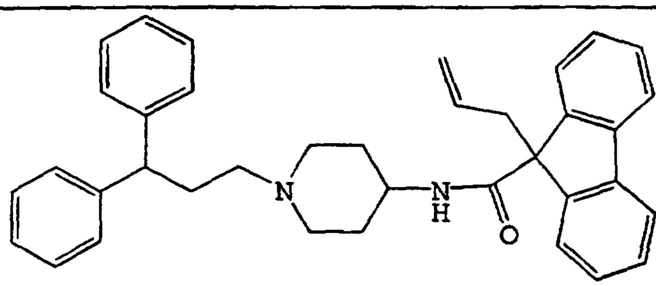 |
| M-6 | 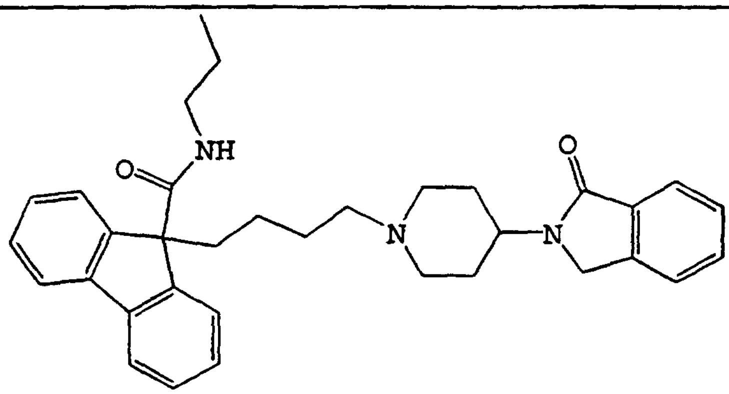 |

| | |
|---|---|
| M-7 | 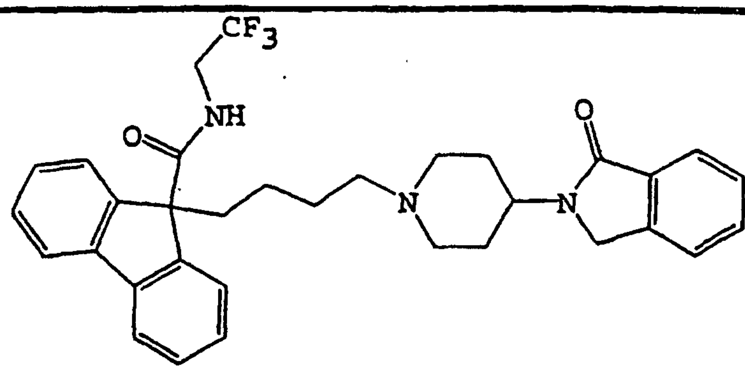 |
| M-8 | 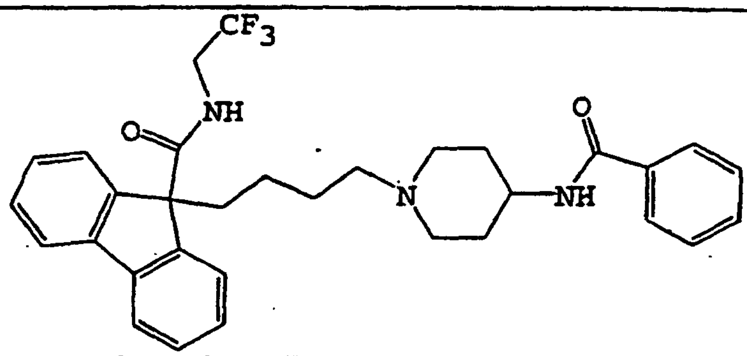 |
| M-9 | 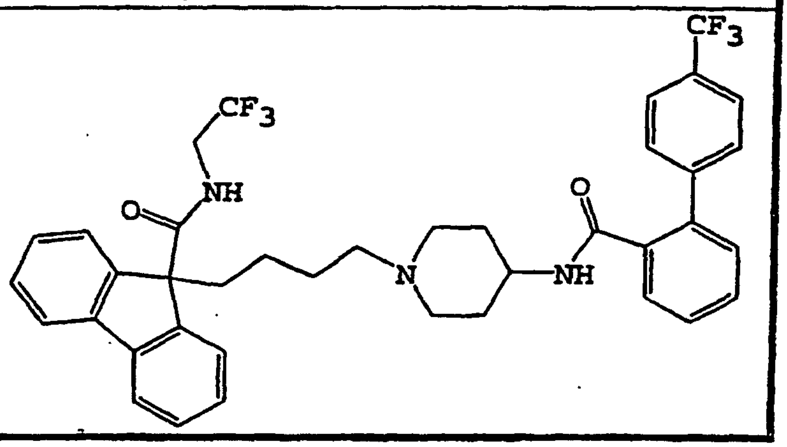 |

In the combination of the present invention, the IBAT inhibitor is preferably a benzothiazepine IBAT inhibitor. In one preferred embodiment, the benzothiazepine IBAT inhibitor is compound B-2. In another preferred embodiment, the benzothiazepine IBAT inhibitor is compound B-7. In yet another preferred embodiment, the IBAT inhibitor is a benzothiepine IBAT inhibitor. Each of the following benzothiepine IBAT inhibitors represents a separate preferred embodiment of the present invention.

B-1.
B-3.
B-4.
B-5.
B-6.
B-8.
B-9.
B-10.
B-11.
B-12.
B-13.

B-14.
B-15.
B-16.
B-17.
B-18.
B-19.
B-21.
B-22.
B-23.
B-24.
B-25.
B-26.
B-27.
B-28.
B-29.
B-30.
B-31.
B-32.
B-33.
B-34.
B-35.
B-36.
B-37.
B-38.
B-39.

In yet another preferred embodiment, the IBAT inhibitor is a naphthalene IBAT inhibitor, for example, compound B-20.

Hypertension is defined as persistently high blood pressure. Generally, adults are classified as being hypertensive when systolic blood pressure is persistently above 140 mmHg or when diastolic blood pressure is above 90 mmHg. Long-term risks for cardiovascular mortality increase in a direct relationship with persistent blood pressure. (E. Braunwald, Heart Disease, 5th ed., W.B. Saunders & Co., Philadelphia, 1997, pp. 807-823.) Blood pressure is a function of cardiac output and peripheral resistance of the vascular system and can be represented by the following equation:

$$BP = CO \times PR$$

wherein BP is blood pressure, CO is cardiac output, and PR is peripheral resistance. (Id., p. 816.) Factors affecting peripheral resistance include obesity and/or functional constriction. Factors affecting cardiac output include venous constriction. Functional constriction of the blood vessels can be caused by a variety of factors including thickening of blood vessel walls resulting in diminishment of the inside diameter of the vessels. Another factor which affects systolic blood pressure is rigidity of the aorta (Id., p. 811.)

Hypertension and atherosclerosis or other hyperlipidemic conditions often coexist in a patient. It is possible that certain hyperlipidemic conditions such as atherosclerosis can have a direct or indirect affect on hypertension. For example, atherosclerosis frequently results in diminishment of the inside diameter of blood vessels. Furthermore, atherosclerosis frequently results in increased rigidity of blood vessels, including the aorta. Both diminished inside diameter of blood vessels and rigidity of blood vessels are factors which contribute to hypertension.

Myocardial infarction is the necrosis of heart muscle cells resulting from oxygen deprivation and is usually caused by an obstruction of the supply of blood to the affected tissue. For example, hyperlipidemia or hypercholesterolemia can cause the formation of atherosclerotic plaques which can cause obstruction of blood flow and thereby cause myocardial infarction. (Id., pp. 1185-1187.) Another major risk factor for myocardial infarction is hypertension. (Id., p. 815.) In other words, hypertension and hyperlipidemic conditions such as atherosclerosis or hypercholesterolemia work in concert to cause myocardial infarction.

Coronary heart disease is another disease which is caused or aggravated by multiple factors including hyperlipidemic conditions and hypertension. Control of both hyperlipidemic conditions and hypertension are important to control symptoms or disease progression of coronary heart disease.

Angina pectoris is acute chest pain which is caused by decreased blood supply to the heart. Decreased blood supply to the heart is known as myocardial ischemia. Angina pectoris can be the result of, for example, stenosis of the

aorta, pulmonary stenosis, and ventricular hypertrophy. Some antihypertensive agents, for example amlodipine, control angina pectoris by reducing peripheral resistance.

It is now disclosed that a therapy which controls hypertension and which in combination controls hyperlipidemic conditions will reduce risk from cardiovascular disease or symptoms of heart disease, for example coronary heart disease, myocardial infarction, or angina pectoris.

Many of the compounds useful in the present invention can have at least two asymmetric carbon atoms, and therefore include racemates and stereoisomers, such as diastereomers and enantiomers, in both pure form and in admixture. Such stereoisomers can be prepared using conventional techniques, either by reacting enantiomeric starting materials, or by separating isomers of compounds of the present invention.

Isomers may include geometric isomers, for example cis-isomers or trans-isomers across a double bond. All such isomers are contemplated among the compounds useful in the present invention.

The compounds useful in the present invention also include tautomers.

The compounds useful in the present invention as discussed below include their salts, solvates and prodrugs.

## Dosages, Formulations, and Routes of Administration

The compositions of the present invention can be administered for the prophylaxis and treatment of hyperlipidemic diseases or conditions by any means, preferably oral, that produce contact of these compounds with their site of action in the body, for example in the ileum of a mammal, e.g., a human.

For the prophylaxis or treatment of the conditions referred to above, the compounds useful in the compositions and methods of the present invention can be used as the compound *per se*. Pharmaceutically acceptable salts are particularly suitable for medical applications because of their greater aqueous solubility relative to the parent compound. Such salts must clearly have a pharmaceutically acceptable anion or cation. Suitable pharmaceutically acceptable acid addition salts of the compounds of the present invention when possible include those derived from inorganic acids, such as hydrochloric, hydrobromic, phosphoric, metaphosphoric, nitric, sulfonic, and sulfuric acids, and organic acids such as acetic, benzenesulfonic, benzoic, citric, ethanesulfonic, fumaric, gluconic, glycolic, isothionic, lactic, lactobionic, maleic, malic, methanesulfonic, succinic, toluenesulfonic, tartaric, and trifluoroacetic acids. The chloride salt is particularly preferred for medical purposes. Suitable pharmaceutically acceptable base salts include ammonium salts, alkali metal salts such as sodium and potassium salts, and alkaline earth salts such as magnesium and calcium salts.

The anions useful in the present invention are, of course, also required to be pharmaceutically acceptable and are also selected from the above list.

The compounds useful in the present invention can be presented with an acceptable carrier in the form of a pharmaceutical composition. The carrier must, of course, be acceptable in the sense of being compatible with the other ingredients of the composition and must not be deleterious to the recipient. The carrier can be a solid or a liquid, or both, and is preferably formulated with the compound as a unit-dose composition, for example, a tablet, which can contain from 0.05% to 95% by weight of the active compound. Other pharmacologically active substances can also be present, including other compounds of the present invention. The pharmaceutical compositions of the invention can be prepared by any of the well known techniques of pharmacy, consisting essentially of admixing the components.

These compounds can be administered by any conventional means available for use in conjunction with pharmaceuticals, either as individual therapeutic compounds or as a combination of therapeutic compounds.

The amount of compound which is required to achieve the desired biological effect will, of course, depend on a number of factors such as the specific compound chosen, the use for which it is intended, the mode of administration, and the clinical condition of the recipient.

In general, a total daily dose of an IBAT inhibitor can be in the range of from about 0.01 to about 1000 mg/day, preferably from about 0.1 mg to about 50 mg/day, more preferably from about 1 to about 10 mg/day.

For MTP inhibitors, a daily dose of about 0.001 to about 800 mg/kg body weight/day, preferably between about 0.01 to about 500 mg/kg body weight/day, more preferably between about 0.1 to about 300 mg/kg body weight/day, and more preferably still between about 1 to about 200 mg/kg body weight/day will generally be appropriate.

The daily doses described in the preceding paragraphs for the various therapeutic compounds can be administered to the patient in a single dose, or in proportionate multiple subdoses. Subdoses can be administered 2 to 6 times per day. Doses can be in sustained release form effective to obtain desired results.

In the case of pharmaceutically acceptable salts, the weights indicated above refer to the weight of the acid equivalent or the base equivalent of the therapeutic compound derived from the salt.

Oral delivery of the combinations of the present invention can include formulations, as are well known in the art, to provide prolonged or sustained delivery of the drug to the gastrointestinal tract by any number of mechanisms. These include, but are not limited to, pH sensitive release from the dosage form based on the changing pH of the small intestine, slow erosion of a tablet or capsule, retention in the stomach based on the physical properties of the formulation, bioadhesion of the dosage form to the mucosal lining of the intestinal tract, or enzymatic release of the active

drug from the dosage form. For some of the therapeutic compounds useful in the present invention (e.g., IBAT inhibitors),

the intended effect is to extend the time period over which the active drug molecule is delivered to the site of action (e.g., the ileum) by manipulation of the dosage form. Thus, enteric-coated and enteric-coated controlled release formulations are within the scope of the present invention. Suitable enteric coatings include cellulose acetate phthalate, polyvinylacetate phthalate, hydroxypropylmethylcellulose phthalate and anionic polymers of methacrylic acid and methacrylic acid methyl ester.

The combinations of the present invention can be delivered orally either in a solid, in a semi-solid, or in a liquid form. When in a liquid or in a semi-solid form, the combinations of the present invention can, for example, be in the form of a liquid, syrup, or contained in a gel capsule (e.g., a gel cap).

When administered intravenously, the dose for an IBAT inhibitor can, for example, be in the range of from about 0.1 mg/kg body weight to about 1.0 mg/kg body weight, preferably from about 0.25 mg/kg body weight to about 0.75 mg/kg body weight, more preferably from about 0.4 mg/kg body weight to about 0.6 mg/kg body weight.

The dose of any of these therapeutic compounds can be conveniently administered as an infusion of from about 10 ng/kg body weight to about 100 ng/kg body weight per minute. Infusion fluids suitable for this purpose can contain, for example, from about 0.1 ng to about 10 mg, preferably from about 1 ng to about 10 mg per milliliter. Unit doses can contain, for example, from about 1 mg to about 10 g of the compound of the present invention. Thus, ampoules for injection can contain, for example, from about 1 mg to about 100 mg.

Pharmaceutical compositions according to the present invention include those suitable for oral, rectal, topical, buccal (e.g., sublingual), and parenteral (e.g., subcutaneous, intramuscular, intradermal, or intravenous) administration, although the most suitable route in any given case will depend on the nature and severity of the condition being treated and on the nature of the particular compound which is being used. In most cases, the preferred route of administration is oral.

Pharmaceutical compositions suitable for oral administration can be presented in discrete units, such as capsules, cachets, lozenges, or tablets, each containing a predetermined amount of at least one therapeutic compound useful in the present invention; as a powder or granules; as a solution or a suspension in an aqueous or non-aqueous liquid; or as an oil-in-water or water-in-oil emulsion. As indicated, such compositions can be prepared by any suitable method of pharmacy which includes the step of bringing into association the active compound(s) and the carrier (which can constitute one or more accessory ingredients). In general, the compositions are prepared by uniformly and intimately admixing the active compound with a liquid or finely divided solid carrier, or both, and then, if necessary, shaping the product. For example, a tablet can be prepared by compressing or molding a powder or granules of the compound, optionally with one or more assessory ingredients. Compressed tablets can be prepared by compressing, in a suitable machine, the compound in a free-flowing form, such as a powder or granules optionally mixed with a binder, lubricant, inert diluent and/or surface active/dispersing agent(s). Molded tablets can be made by molding, in a suitable machine, the powdered compound moistened with an inert liquid diluent.

Pharmaceutical compositions suitable for buccal (sublingual) administration include lozenges comprising a compound of the present invention in a flavored base, usually sucrose, and acacia or tragacanth, and pastilles comprising the compound in an inert base such as gelatin and glycerin or sucrose and acacia.

Pharmaceutical compositions suitable for parenteral administration conveniently comprise sterile aqueous preparations of a compound of the present invention. These preparations are preferably administered intravenously, although administration can also be effected by means of subcutaneous, intramuscular, or intradermal injection. Such preparations can conveniently be prepared by admixing the compound with water and rendering the resulting solution sterile and isotonic with the blood. Injectable compositions according to the invention will generally contain from 0.1 to 5% w/w of a compound disclosed herein.

Pharmaceutical compositions suitable for rectal administration are preferably presented as unit-dose suppositories. These can be prepared by admixing a compound of the present invention with one or more conventional solid carriers, for example, cocoa butter, and then shaping the resulting mixture.

Pharmaceutical compositions suitable for topical application to the skin preferably take the form of an ointment, cream, lotion, paste, gel, spray, aerosol, or oil. Carriers which can be used include petroleum jelly (e.g., Vaseline), lanolin, polyethylene glycols, alcohols, and combinations of two or more thereof. The active compound is generally present at a concentration of from 0.1 to 50% w/w of the composition, for example, from 0.5 to 2%.

Transdermal administration is also possible. Pharmaceutical compositions suitable for transdermal administration can be presented as discrete patches adapted to remain in intimate contact with the epidermis of the recipient for a prolonged period of time. Such patches suitably contain a compound of the present invention in an optionally buffered, aqueous solution, dissolved and/or dispersed in an adhesive, or dispersed in a polymer. A suitable concentration of the active compound is about 1% to 35%, preferably about 3% to 15%. As one particular possibility, the compound can be delivered from the patch by electrotransport or iontophoresis, for example, as described in Pharmaceutical Research, 3(6), 318 (1986).

In any case, the amount of active ingredient that can be combined with carrier materials to produce a single dosage form to be administered will vary depending upon the host treated and the particular mode of administration.

The solid dosage forms for oral administration including capsules, tablets, pills, powders, gel caps, and granules noted above comprise one or more compounds useful in the present invention admixed with at least one inert diluent such as sucrose, lactose, or starch. Such dosage forms may also comprise, as in normal practice, additional substances other than inert diluents, e.g., lubricating agents such as magnesium stearate or solubilizing agents such as cyclodextrins. In the case of capsules, tablets, powders, granules, gel caps, and pills, the dosage forms may also comprise buffering agents. Tablets and pills can additionally be prepared with enteric coatings.

Liquid dosage forms for oral administration can include pharmaceutically acceptable emulsions, solutions, suspensions, syrups, and elixirs containing inert diluents commonly used in the art, such as water. Such compositions may also comprise adjuvants, such as wetting agents, emulsifying and suspending agents, and sweetening, flavoring, and perfuming agents.

Injectable preparations, for example, sterile injectable aqueous or oleaginous suspensions may be formulated according to the known art using suitable dispersing or setting agents and suspending agents. The sterile injectable preparation may also be a sterile injectable solution or suspension in a nontoxic parenterally acceptable diluent or solvent, for example, as a solution in 1,3-butanediol. Among the acceptable vehicles and solvents that may be employed are water, Ringer's solution, and isotonic sodium chloride solution. In addition, sterile, fixed oils are conventionally employed as a solvent or suspending medium. For this purpose any bland fixed oil may be employed including synthetic mono- or diglycerides. In addition, fatty acids such as oleic acid find use in the preparation of injectables.

Pharmaceutically acceptable carriers encompass all the foregoing and the like.

In combination therapy, administration of two or more of the therapeutic agents useful in the present invention may take place sequentially in separate formulations, or may be accomplished by simultaneous administration in a single formulation or separate formulations. Administration may be accomplished by oral route, or by intravenous, intramuscular, or subcutaneous injections. The formulation may be in the form of a bolus, or in the form of aqueous or non-aqueous isotonic sterile injection solutions or suspensions. These solutions and suspensions may be prepared from sterile powders or granules having one or more pharmaceutically-acceptable carriers or diluents, or a binder such as gelatin or hydroxypropylmethyl cellulose, together with one or more of a lubricant, preservative, surface active or dispersing agent.

For oral administration, the pharmaceutical composition may be in the form of, for example, a tablet, capsule, suspension, or liquid. Capsules, tablets, etc., can be prepared by conventional methods well known in the art. The pharmaceutical composition is preferably made in the form of a dosage unit containing a particular amount of the active ingredient or ingredients. Examples of dosage units are tablets or capsules. These may with advantage contain one or more therapeutic compound in an amount described above. For example, in the case of an IBAT inhibitor, the dose range may be from about 0.01 mg/day to about 500 mg/day or any other dose, dependent upon the specific inhibitor, as is known in the art.

The active ingredients may also be administered by injection as a composition wherein, for example, saline, dextrose, or water may be used as a suitable carrier. A suitable daily dose of each active therapeutic compound is one that achieves the same blood serum level as produced by oral administration as described above.

The therapeutic compounds may further be administered by any combination of oral/oral, oral/parenteral, or parenteral/parenteral route.

Pharmaceutical compositions for use in the treatment methods of the present invention may be administered in oral form or by intravenous administration. Oral administration of the combination therapy is preferred. Dosing for oral administration may be with a regimen calling for single daily dose, or for a single dose every other day, or for multiple, spaced doses throughout the day. The therapeutic compounds which make up the combination therapy may be administered simultaneously, either in a combined dosage form or in separate dosage forms intended for substantially simultaneous oral administration. The therapeutic compounds which make up the combination therapy may also be administered sequentially, with either therapeutic compound being administered by a regimen calling for two-step ingestion. Thus, a regimen may call for sequential administration of the therapeutic compounds with spaced-apart ingestion of the separate, active agents. The time period between the multiple ingestion steps may range from a few minutes to several hours, depending upon the properties of each therapeutic compound such as potency, solubility, bioavailability, plasma half-life and kinetic profile of the therapeutic compound, as well as depending upon the effect of food ingestion and the age and condition of the patient. Circadian variation of the target molecule concentration may also determine the optimal dose interval. The therapeutic compounds of the combined therapy whether administered simultaneously, substantially simultaneously, or sequentially, may involve a regimen calling for administration of one therapeutic compound by oral route and another therapeutic compound by intravenous route. Whether the therapeutic compounds of the combined therapy are administered by oral or intravenous route, separately or together, each such therapeutic compound will be contained in a suitable pharmaceutical formulation of pharmaceutically-acceptable excipients, diluents or other formulations components. Examples of suitable pharmaceutically-acceptable formulations

containing the therapeutic compounds for oral administration are given above.

**Treatment Regimen**

The dosage regimen to prevent, give relief from, or ameliorate a disease condition having hyperlipemia as an element of the disease, e.g., atherosclerosis, or to protect against or treat further high cholesterol plasma or blood levels with the compounds and/or compositions of the present invention is selected in accordance with a variety of factors. These include the type, age, weight, sex, diet, and medical condition of the patient, the severity of the disease, the route of administration, pharmacological considerations such as the activity, efficacy, pharmacokinetics and toxicology profiles of the particular compound employed, whether a drug delivery system is utilized, and whether the compound is administered as part of a drug combination. Thus, the dosage regimen actually employed may vary widely and therefore deviate from the preferred dosage regimen set forth above.

Initial treatment of a patient suffering from a hyperlipidemic condition can begin with the dosages indicated above. Treatment should generally be continued as necessary over a period of several weeks to several months or years until the hyperlipidemic disease condition has been controlled or eliminated. Patients undergoing treatment with the compounds or compositions disclosed herein can be routinely monitored by, for example, measuring serum LDL and total cholesterol levels by any of the methods well known in the art, to determine the effectiveness of the combination therapy. Continuous analysis of such data permits modification of the treatment regimen during therapy so that optimal effective amounts of each type of therapeutic compound are administered at any point in time, and so that the duration of treatment can be determined as well. In this way, the treatment regimen/dosing schedule can be rationally modified over the course of therapy so that the lowest amount of the therapeutic compounds which together exhibit satisfactory effectiveness is administered, and so that administration is continued only so long as is necessary to successfully treat the hyperlipidemic condition.

A potential advantage of the combination disclosed herein may be reduction of the amount of any individual therapeutic compound, or all therapeutic compounds, effective in treating hyperlipidemic conditions such as atherosclerosis and hypercholesterolemia.

One of the several embodiments of the present invention provides a combination comprising the use of a first amount of an IBAT inhibitor and a second amount of a microsomaltriglyceride transfer protein inhibiting compound for the manufacture of a medicament for prophylaxis or treatment of hyperlipidemia or atherosclerosis, wherein the first and second amounts together comprise an anti-hyperlipidemic condition effective amount or an anti-atherosclerotic condition effective amount of the compounds.

In a preferred embodiment, the IBAT inhibitor is a benzothiepine IBAT inhibitor. In another preferred embodiment, the IBAT inhibitor is a benzothiazepine IBAT inhibitor. In yet another preferred embodiment, the IBAT inhibitor is a naphthalene IBAT inhibitor.

The embodiments of the present invention can comprise a combination therapy using two or more of the therapeutic compounds described or incorporated herein. The combination therapy can comprise two or more therapeutic compounds from different classes of chemistry,

Therapeutic combinations can comprise more than two therapeutic compounds. For example, two or more therapeutic compounds from the same class of chemistry can comprise the therapy, e.g. a combination therapy comprising two or more IBAT inhibitors.

A further embodiment of the instant invention comprises the use of any of the cardiovascular combination therapies described herein for the prophylaxis or treatment of hypercholesterolemia or atherosclerosis.

The following non-limiting examples serve to illustrate various aspects of the present invention.

Any of the MTP inhibitor compounds described by Wetterau et al. (Id.) can be used in combinations of the present invention wherein the combination comprises a first amount of an ileal bile acid transporter inhibiting compound and a second amount of a MTP inhibitor wherein the first and second amounts together comprise an anti-hyperlipidemic condition effective amount, an anti-atherosclerotic condition effective amount, an anti-hypercholesterolemic condition effective amount, or an anti-hypertensive condition effective amount of the compounds. The IBAT inhibitor in the embodiments of this invention is preferably a benzothiepine IBAT inhibitor. In another preferred embodiment, the IBAT inhibitor is a benzothiazepine IBAT inhibitor. In still another preferred embodiment, the IBAT inhibitor is a naphthalene IBAT inhibitor. The IBAT inhibitor can, without limitation, be any one or combination of the compounds listed in Table 1.

In another embodiment the present invention provides a method for the prophylaxis or treatment of a hyperlipidemic condition or disorder in a mammal which comprises administering a first amount of an ileal bile acid transport inhibitor compound and a second amount of a microsomal triglyceride transfer protein inhibiting compound wherein the first amount and the second amount together comprise an anti-hyperlipidemic condition effective amount, an anti-atherosclerotic condition effective amount, or an anti-hypercholesterolemic condition effective amount of the compounds.

In another embodiment the present invention provides a kit for achieving a therapeutic effect in a mammal

comprising an amount of an ileal bile acid transport inhibiting compound in a first unit dosage form; an amount of a microsomal triglyceride transfer protein inhibiting compound in a second unit dosage form; and container means for containing said first and second unit dosage forms.

## BIOLOGICAL ASSAYS

The utility of the combinations of the present invention can be shown by the following assays. These assays are performed *in vitro* and in animal models essentially using procedures recognized to show the utility of the present invention.
Assays with compounds not falling under the scope of the claims are for comparative purposes.

### In Vitro Assay of compounds that inhibit IBAT-mediated uptake of [$^{14}$C]-Taurocholate (TC) in H14 Cells

Baby hamster kidney cells (BHK) transfected with the cDNA of human IBAT (H14 cells) are to be seeded at 60,000 cells/well in 96 well Top-Count tissue culture plates for assays run within in 24 hours of seeding, 30,000 cells/well for assays run within 48 hours, and 10,000 cells/well for assays run within 72 hours.

On the day of assay, the cell monolayer is gently washed once with 100 µl assay buffer (Dulbecco's Modified Eagle's medium with 4.5 g/L glucose + 0.2% (w/v) fatty acid free bovine serum albumin- (FAF)BSA). To each well 50 µl of a two-fold concentrate of test compound in assay buffer is added along with 50 µl of 6 µM [$^{14}$C]-taurocholate in assay buffer (final concentration of 3 µM [$^{14}$C]-taurocholate). The cell culture plates are incubated 2 hours at 37°C prior to gently washing each well twice with 100 µl 4°C Dulbecco's phosphate-buffered saline (PBS) containing 0.2% (w/v) (FAF)BSA. The wells are then to be gently washed once with 100 µl 4°C PBS without (FAF)BSA. To each 200 µl of liquid scintillation counting fluid is to be added, the plates are heat sealed and shaken for 30 minutes at room temperature prior to measuring the amount of radioactivity in each well on a Packard Top-Count instrument.

### *In Vitro* Assay of compounds that inhibit uptake of [$^{14}$C]-Alanine

The alanine uptake assay can be performed in an identical fashion to the taurocholate assay, with the exception that labeled alanine is to be substituted for the labeled taurocholate.

### *In Vivo* Assay of compounds that inhibit Rat Ileal uptake of [$^{14}$C]-Taurocholate into Bile

(See "Metabolism of 3α,7β-dihydroxy-7α-methyl-5β-cholanoic acid and 3α,7β-dihydroxy-7α-methyl-5β-cholanoic acid in hamsters" in Biochimica et Biophysica Acta, 833, 196-202 (1985) by Une et al.).

Male wistar rats (200-300 g) are to be anesthetized with inactin @100 mg/kg. Bile ducts are cannulated with a 10" length of PE10 tubing. The small intestine is exposed and laid out on a gauze pad. A canulae (1/8" luer lock, tapered female adapter) is inserted at 12 cm from the junction of the small intestine and the cecum. A slit is cut at 4 cm from this same junction (utilizing a 8 cm length of ileum). 20 ml of warm Dulbecco's phosphate buffered saline, pH 6.5 (PBS) is used to flush out the intestine segment. The distal opening is cannulated with a 20 cm length of silicone tubing (0.02" I.D. x 0.037" O.D.). The proximal cannulae is hooked up to a peristaltic pump and the intestine is washed for 20 min with warm PBS at 0.25 ml/min. Temperature of the gut segment is to be monitored continuously. At the start of the experiment, 2.0 ml of control sample ([$^{14}$C]-taurocholate @ 0.05 mCi/ml with 5 mM non-radiolabeled taurocholate) is loaded into the gut segment with a 3 ml syringe and bile sample collection is begun. Control sample is infused at a rate of 0.25 ml/min for 21 min. Bile samples fractions will be collected every 3 minute for the first 27 minutes of the procedure. After the 21 min of sample infusion, the ileal loop is washed out with 20 ml of warm PBS (using a 30 ml syringe), and then the loop is washed out for 21 min with warm PBS at 0.25 ml/min. A second perfusion is to be initiated as described above but with test compound being administered as well (21 min administration followed by 21 min of wash out) and bile to be sampled every 3 min for the first 27 min. If necessary, a third perfusion will be performed as above that typically contains the control sample.

### Measurement of Hepatic Cholesterol Concentration (HEPATIC CHOL)

Liver tissue is to be weighed and homogenized in chloroform:methanol (2:1). After homogenization and centrifugation the supernatant is separated and dried under nitrogen. The residue is to be dissolved in isopropanol and the cholesterol content will be measured enzymatically, using a combination of cholesterol oxidase and peroxidase, as described by Allain, C. A. et al., Clin. Chem., 20, 470 (1974).

**Determination of Serum Cholesterol (SER.CHOL, HDL-CHOL, TGI and VLDL + LDL)**

Total serum cholesterol (SER.CHOL) are to be measured enzymatically using a commercial kit from Wako Fine Chemicals (Richmond, VA); Cholesterol C11, Catalog No. 276-64909. HDL cholesterol (HDL-CHOL) will be assayed using this same kit after precipitation of VLDL and LDL with Sigma Chemical Co. HDL Cholesterol reagent, Catalog No. 352-3 (dextran sulfate method). Total serum triglycerides (blanked) (TGI) will be assayed enzymatically with Sigma Chemical Co. GPO-Trinder, Catalog No. 337-B. VLDL and LDL (VLDL + LDL) cholesterol concentrations will be calculated as the difference between total and HDL cholesterol.

**Measurement of Hepatic Cholesterol 7-α-Hydroxylase Activity (7a-OHase)**

Hepatic microsomes are to be prepared by homogenizing liver samples in a phosphate/sucrose buffer, followed by centrifugal separation. The final pelleted material is resuspended in buffer and an aliquot will be assayed for cholesterol 7-α-hydroxylase activity by incubating for 5 minutes at 37° C in the presence of NADPH. Following extraction into petroleum ether, the organic solvent is evaporated and the residue is dissolved in acetonitrile/methanol. The enzymatic product will be separated by injecting an aliquot of the extract onto a $C_{18}$ reversed phase HPLC column and quantitating the eluted material using UV detection at 240nm. (Reference: Horton, J. D., et al. (1994) *J. Clin. Invest.* **93,** 2084).

**Rat Gavage Assay**

Male Wister rats (275-300g) are to be administered IBAT inhibitors using an oral gavage procedure. Drug or vehicle (0.2% TWEEN 80 in water) is administered once a day (9:00-10:0 a.m.) for 4 days at varying dosages in a final volume of 2 mL per kilogram of body weight. (TWEEN 80 is a 20 molar polyethyleneoxide sorbitan monooleate surfactant manufactured by ICI Specialty Chemicals, Wilmington, Delaware, U.S.A.) Total fecal samples are collected during the final 48 hours of the treatment period and analyzed for bile acid content using an enzymatic assay as described below. Compound efficacy will be determined by comparison of the increase in fecal bile acid (FBA) concentration in treated rats to the mean FBA concentration of rats in the vehicle group.

**Measurement of Fecal Bile Acid Concentration (FBA)**

Total fecal output from individually housed rats is to be collected for 24 or 48 hours, dried under a stream of nitrogen, pulverized and weighed. Approximately 0.1 gram is weighed out and extracted into an organic solvent (butanol/water). Following separation and drying, the residue is dissolved in methanol and the amount of bile acid present will be measured enzymatically using the 3α-hydroxysteroid steroid dehydrogenase reaction with bile acids to reduce NAD. (*see* Mashige, F. et al. Clin. Chem., 27, 1352 (1981)).

**[$^3$H]taurocholate Uptake in Rabbit Brush Border Membrane Vesicles (BBMV)**

Rabbit Ileal brush border membranes are to be prepared from frozen ileal mucosa by the calcium precipitation method describe by Malathi *et al.* (Biochimica Biophysica Acta, 554, 259 (1979)). The method for measuring taurocholate is essentially as described by Kramer *et al.* (Biochimica Biophysica Acta, 1111, 93 (1992)). except the assay volume will be 200 μl instead of 100 μl. Briefly, at room temperature a 190 μl solution containing 2μM [$^3$H]-taurocholate (0.75 μCi), 20 mM tris, 100 mM NaCl, 100 mM mannitol pH 7.4 is incubated for 5 sec with 10 μl of brush border membrane vesicles (60-120 μg protein). The incubation is initiated by the addition of the BBMV while vortexing and the reaction is to be stopped by the addition of 5 ml of ice cold buffer (20 mM Hepes-tris, 150 mM KCl) followed immediately by filtration through a nylon filter (0.2 μm pore) and an additional 5 ml wash with stop buffer.

**Acyl-CoA; Cholesterol Acyl Transferase (ACAT)**

Hamster liver and rat intestinal microsomes are to be prepared from tissue as described previously (J. Biol. Chem., 255, 9098 (1980)) and used as a source of ACAT enzyme. The assay will consist of a 2.0 ml incubation containing 24 μM Oleoyl-CoA (0.05 μCi) in a 50 mM sodium phosphate, 2 mM DTT ph 7.4 buffer containing 0.25 % BSA and 200 μg of microsomal protein. The assay will be initiated by the addition of oleoyl-CoA. The reaction proceeds for 5 min at 37° C and will be terminated by the addition of 8.0 ml of chloroform/ methanol (2:1). To the extraction is added 125 μg of cholesterol oleate in chloroform methanol to act as a carrier and the organic and aqueous phases of the extraction are separated by centrifugation after thorough vortexing. The chloroform phase is to be taken to dryness and then spotted on a silica gel 60 TLC plate and developed in hexane/ethyl ether (9:1). The amount of cholesterol

ester formed will be determined by measuring the amount of radioactivity incorporated into the cholesterol oleate spot on the TLC plate with a Packard Instaimager.

**Dog Model for Evaluating Lipid Lowering Drugs**

Male beagle dogs, obtained from a vendor such as Marshall farms and weighing 6-12 kg are fed once a day for two hours and given water ad libitum. Dogs may be randomly assigned to a dosing groups consisting of 6 to 12 dogs each, such as: vehicle, i.g.; 1mg/kg, i.g.; 2mg/kg, i.g.; 4 mg/kg, i.g.; 2 mg/kg, p.o. (powder in capsule). Intragastric dosing of a therapeutic material dissolved in aqueous solution (for example, 0.2% Tween 80 solution [polyoxyethylene mono-oleate, Sigma Chemical Co., St. Louis, MO]) may be done using a gavage tube. Prior to initiating dosing, blood samples may be drawn from the cephalic vein in the morning before feeding in order to evaluate serum cholesterol (total and HDL) and triglycerides. For several consecutive days animals are dosed in the morning, prior to feeding. Animals are to be allowed 2 hours to eat before any remaining food is removed. Feces are to be collected over a 2 day period at the end of the study and may be analyzed for bile acid or lipid content. Blood samples are also to be taken, at the end of the treatment period, for comparison with pre-study serum lipid levels. Statistical significance will be determined using the standard student's T-test with $p<.05$.

**Dog Serum Lipid Measurement**

Blood is to be collected from the cephalic vein of fasted dogs in serum separator tubes (Vacutainer SST, Becton Dickinson and Co., Franklin Lakes, NJ). The blood is centrifuged at 2000 rpm for 20 minutes and the serum decanted.

Total cholesterol may be measured in a 96 well format using a Wako enzymatic diagnostic kit (Cholesterol CII) (Wako Chemicals, Richmond, VA), utilizing the cholesterol oxidase reaction to produce hydrogen peroxide which is measured colorimetrically. A standard curve from 0.5 to 10 µg cholesterol is to be prepared in the first 2 columns of the plate. The serum samples (20-40 µl, depending on the expected lipid concentration) or known serum control samples are added to separate wells in duplicate. Water is added to bring the volume to 100 µl in each well. A 100 µl aliquot of color reagent is added to each well and the plates will be read at 500 nm after a 15 minute incubation at 37 degrees centigrade.

HDL cholesterol may be assayed using Sigma kit No. 352-3 (Sigma Chemical Co., St. Louis, MO) which utilizes dextran sulfate and Mg ions to selectively precipitate LDL and VLDL. A volume of 150 µl of each serum sample is to be added to individual microfuge tubes, followed by 15 µl of HDL cholesterol reagent (Sigma 352-3). Samples are to be mixed and centrifuged at 5000 rpm for 5 minutes. A 50 µl aliquot of the supernatant is to be then mixed with 200 µl of saline and assayed using the same procedure as for total cholesterol measurement.

Triglycerides are to be measured using Sigma kit No. 337 in a 96 well plate format. This procedure will measure glycerol, following its release by reaction of triglycerides with lipoprotein lipase. Standard solutions of glycerol (Sigma 339-11) ranging from 1 to 24 µg are to be used to generate the standard curve. Serum samples (20-40 µl, depending on the expected lipid concentration) are added to wells in duplicate. Water is added to bring the volume to 100 µl in each well and 100 µl of color reagent was also added to each well. After mixing and a 15 minute incubation, the plates will be read at 540 nm and the triglyceride values calculated from the standard curve. A replicate plate is also to be run using a blank enzyme reagent to correct for any endogenous glycerol in the serum samples.

**Dog Fecal Bile Acid Measurement**

Fecal samples may be collected to determine the fecal bile acid (FBA) concentration for each animal. Fecal collections may be made during the final 48 hours of the study, for two consecutive 24 hour periods between 9:00 am and 10:00 am each day, prior to dosing and feeding. The separate two day collections from each animal are to be weighed, combined and homogenized with distilled water in a processor (Cuisinart) to generate a homogeneous slurry. About 1.4 g of the homogenate is to be extracted in a final concentration of 50% tertiary butanol/distilled water (2:0.6) for 45 minutes in a 37°C water bath and centrifuged for 13 minutes at 2000 x g. The concentration of bile acids (mmoles/day) may be determined using a 96-well enzymatic assay system (1,2). A 20 µl aliquot of the fecal extract is to be added to two sets each of triplicate wells in a 96-well assay plate. A standardized sodium taurocholate solution and a standardized fecal extract solution (previously made from pooled samples and characterized for its bile acid concentration) will also analyzed for assay quality control. Twenty-microliter aliquots of sodium taurocholate, serially diluted to generate a standard curve are similarly to be added to two sets of triplicate wells. A 230 µl reaction mixture containing 1M hydrazine hydrate, 0.1 M pyrophosphate and 0.46 mg/ml NAD is to be added to each well. A 50 µl aliquot of 3a-hydroxysteroid dehydrogenase enzyme (HSD; 0.8 units/ml) or assay buffer (0.1 M sodium pyrophosphate) are then added to one of the two sets of triplicates. All reagents may be obtained from Sigma Chemical Co., St. Louis, MO.

Following 60 minutes of incubation at room temperature, the optical density at 340nm will be measured and the mean of each set of triplicate samples will be calculated. The difference in optical density ± HSD enzyme is to be used to determine the bile acid concentration (mM) of each sample based on the sodium taurocholate standard curve. The bile acid concentration of the extract, the weight of the fecal homogenate (grams) and the body weight of the animal are to be used to calculate the corresponding FBA concentration in mmoles/kg/day for each animal. The mean FBA concentration (mmoles/kg/day) of the vehicle group is to be subtracted from the FBA concentration of each treatment group to determine the increase (delta value) in FBA concentration as a result of the treatment.

## Saponification and Extraction of Neutral Sterols in Hamster Feces

Generally, asample of dried animal feces will be directly saponified with 0.3N KOH/Methanol for 1 hour. After saponification, the samples were filtered to remove solid matter. The samples are extracted twice with petroleum ether, and the extracts are combined and evaporated to dryness with heating under a stream of nitrogen gas. The sample can be analyzed by a Hewlett Packard Model 6890 GC with autosampler using a 50 meter HP-5 Ultra-2 capillary column, 0.33 um film thickness, 0.32 ID, 100:1 split ratio, and an FID detector.

For preparation of the saponified samples, each 0.25 gram sample of dried powdered feces is transferred to a labeled 20 x 150 millimeter screw top tube. Three milliliters of 0.3N KOH/MEOH (7.5 ml of 8N (45%) KOH qs 200 ml with HPLC grade methanol) and 25 microliters of 20mg/ml 5-alpha Cholestane as the internal standard are added to the tubes. The tubes are tightly capped and vortexed. The tubes are placed in a Reacti-Therm heating block in a hood and heated at 70°C for one hourwith intermittent mixing.

For preparation of saponified standards, each standard stock is mixed with 3 milliliters of 0.3N KOH/MEOH and 25 microliters of 5-alpha Cholestane. The standards are capped, heated for one hour at 70 degrees C and extracted. Standard 1 will include a combination of 40 microliters of 20mg/ml Stocks of each of stigmasterol, coprostanol and beta-sitosterol. Standard 2 will be a combination of one microliter of 20mg/ml cholesterol (0.04 ug/ul) and 5 microliters of 20 mg/ml sitostanol (0.2 ug/ul). Standard 3 will be a combination of 40 microliters of 20 mg/ml cholesterol (1.6 ug/ul) and 200 microliters of 20 mg/ml sitostanol (8.0 ug/ul).

For preparation of non-saponified standards, the standards are pipetted into one milliliter V-vials and 25 microliters of 5-alpha cholestane is added. The standards are evaporated to dryness in the Reacti-Therm heating block, removed from the block and allowed to cool. Methylene chloride (500 ul) is added. The extracts are mixed and filtered through the Whatman Anatop filters. Standard 1 will include the combination of 40 microliters of 20 mg/ml stocks of each stigmasterol, coprostanol and beta-sitosterol. Standard 2 will include the combination of 5 microliters of 20mg/ml cholesterol (0.2 ug/ul) and 25 microliters of 20 mg/ml of sitostanol (1.0ug/ul). Standard 3 will include the combination of 20 microliters of 20mg/ml cholesterol (0.8 ug/ul) and 100 microliters of 20 mg/ml sitostanol (4.0 ug/ul). Standard 4 will include the combination of 80 microliters of 20 mg/ml cholesterol (3.2 ug/ul) and 300 microliters of 20 mg/ml sitostanol (12.0 ug/ul).

All tubes are removed from the heating blocks and cooled. Each saponified sample and standard is filtered through a Whatman Autovial Syingeless Filter Device, 0.45um, PTFE (Teflon) membrane. Each tube is washed with 10 mL of petroleum ether, vortexed and combined in the filtering device. The plunger is pushed to collect the sample in a clean 50 mL glass tube. Additional petroleum ether (10 mL) is added to the sample in the 50 mL tube along with 2 mL of water. Each sample is vortexed at a moderate speed (mixing too fast will cause emulsions to form) for 20 seconds. After the layers separated, 2 x 7 mL of the petroleum ether phase is removed and transfered to 16 x 125 millimeter glass tubes. The samples are extracted one more time with the addition of 10 mL of petroleum ether and 8 mL were removed, combining the extracts of each sample. All tubes are evaporated to dryness under a stream of nitrogen gas at 70°C. The residue of each sample is quantitatively transferred to 1.5 mL glass conical vials using 3 x 0.5 mL washes of petroleum ether. The samples are once again evaporated to dryness. After the vials cool to room temperature, 500 microliters of methylene chloride are added. All samples and standards are filtered through Whatman Anotop 10 Plus (0.2um, 10mm) syringe filters. Sufficient filtrate (approximately 300 microliters) is collected into footed micro GC sample tubes. The footed micro tubes are placed in screw capped vials and tightened firmly. Analysis will be by the Hewlett Packard GC procedure.

## CETP ACTIVITY ASSAY IN HUMAN PLASMA (Tritiated cholesteryl ester)

Blood is to be obtained from healthy volunteers. Blood is collected in tubes containing EDTA (EDTA plasma pool). The EDTA human plasma pool previously stored at -20°C, is to be thawed at room temperature, and centrifuged for 5 minutes to remove any particulate matter. Tritiated HDL, radiolabeled in the cholesteryl ester moiety ([$^3$H]CE-HDL) as described by Morton and Zilversmit (J. Biol. Chem., 256, 11992-95 (1981)), is to be added to the plasma to a final concentration of (25 μg/ml cholesterol). Inhibitor compounds are to be added to the plasma as follows: Equal volumes of the plasma containing the [$^3$H]CE-HDL (396 μl) are added by pipette into micro tubes (Titertube®, Bio-Rad laboratories, Hercules, CA). Compounds, usually dissolved as 20-50 mM stock solutions in DMSO, are to be serially diluted in DMSO (or an alternative solvent in some cases, such as dimethylformamide or ethanol). Four µl of each of the serial dilutions of inhibitor compounds or DMSO alone are then added to each of the plasma tubes. The tubes are immediately mixed. Triplicate aliquots (100 µl) from each plasma tube are then transferred to wells of 96-well round-bottomed polystyrene microtiter plates (Corning, Corning, NY). Plates are sealed with plastic film and incubated at 37°C for 4 hours. Test wells are to contain plasma with dilutions of inhibitor compounds. Control wells are to contain plasma with DMSO alone. Blank wells are to contain plasma with DMSO alone that are left in the micro tubes at 4°C for the 4 hour incubation and are added to the microtiter wells at the end of the incubation period. VLDL and LDL are precipitated by the addition of 10 µl of precipitating reagent (1% (w/v) dextran sulfate (Dextralip50)/0.5 M magnesium chloride, pH 7.4) to all wells. The wells are mixed on a plate mixer and then incubated at ambient temperature for 10 min. The plates are then centrifuged at 1000 x g for 30 min at 10°C. The supernatants (50 µl) from each well are then transferred to Picoplate™ 96 plate wells (Packard, Meriden, CT) containing 250:1 Microscint™-40 (Packard, Meriden, CT). The plates are heat-sealed (TopSeal™-P, Packard, Meriden, CT) according to the manufacturer's directions and mixed for 30 min. Radioactivity will be measured on a microplate scintillation counter (TopCount, Packard, Meriden, CT). $IC_{50}$ values will be determined as the concentration of inhibitor compound inhibiting transfer of [$^3$H]CE from the supernatant [$^3$H] CE-HDL to the precipitated VLDL and LDL by 50% compared to the transfer obtained in the control wells. The maximum percentage transfer (in the control wells) will be determined using the following equation:

$$\% \text{ Transfer} = \frac{[dpm_{blank} - dpm_{control}] \times 100}{dpm_{blank}}$$

The percentage of control transfer determined in the wells containing inhibitor compounds is determined as follows:

$$\% \text{ Control} = \frac{[dpm_{blank} - dpm_{test}] \times 100}{dpm_{blank} - dpm_{control}}$$

$IC_{50}$ values will be calculated from plots of % control

versus concentration of inhibitor compound.

**CETP Activity *In Vitro***

The ability of compounds to inhibit CETP activity are assessed using an *in vitro* assay that measures the rate of transfer of radiolabeled cholesteryl ester ([$^3$H]CE) from HDL donor particles to LDL acceptor particles. Details of the assay are provided by Glenn et al. (Glenn and Melton, "Quantification of Cholesteryl Ester Transfer Protein (CETP): A) CETP Activity and B) Immunochemical Assay of CETP Protein," Meth. Enzymol., 263, 339-351 (1996)). CETP can be obtained from the serum-free conditioned medium of CHO cells transfected with a cDNA for CETP (Wang, S. et al. J. Biol. Chem. 267, 17487-17490 (1992)). To measure CETP activity, [$^3$H]CE-labeled HDL, LDL, CETP and assay buffer (50 mM tris(hydroxymethyl)aminomethane, pH 7.4; 150 mM sodium chloride; 2 mM ethylenediamine-tetraacetic acid; 1% bovine serum albumin) are incubated in a volume of 200 µl, for 2 hours at 37°C in 96 well plates. LDL is differentially precipitated by the addition of 50 µl of 1% (w/v) dextran sulfate/0.5 M magnesium chloride, mixed by vortex, and incubated at room temperature for 10 minutes. The solution (200 µl) is transferred to a filter plate (Millipore). After filtration, the radioactivity present in the precipitated LDL is measured by liquid scintillation counting. Correction for non-specific transfer or precipitation is made by including samples that do not contain CETP. The rate of [$^3$H]CE transfer using this assay is linear with respect to time and CETP concentration, up to 25-30% of [$^3$H]CE transferred.

The potency of test compounds can be determined by performing the above described assay in the presence of varying concentrations of the test compounds and determining the concentration required for 50% inhibition of transfer of [$^3$H]CE from HDL to LDL. This value is defined as the $IC_{50}$. The $IC_{50}$ values determined from this assay will be accurate when the $IC_{50}$ is greater than 10 nM. In the case where compounds have greater inhibitory potency, accurate measurements of $IC_{50}$ may be determined using longer incubation times (up to 18 hours) and lower final concentrations of CETP (< 50 nM).

**Inhibition of CETP Activity In Vivo.**

Inhibition of CETP activity by a test compound can be determined by administering the compound to an animal

by intravenous injection or oral gavage, measuring the amount of transfer of tritium-labeled cholesteryl ester ([$^3$H]CE) from HDL to VLDL and LDL particles, and comparing this amount of transfer with the amount of transfer observed in control animals.

Male golden Syrian hamsters are to be maintained on a diet of chow containing 0.24% cholesterol for at least two weeks prior to the study. For animals receiving intravenous dosing, immediately before the experiment, animals are anesthetized with pentobarbital. Anesthesia is maintained throughout the experiment. In-dwelling catheters are to be inserted into the jugular vein and carotid artery. At the start of the experiment all animals will receive 0.2 ml of a solution containing [$^3$H]CE-HDL into the jugular vein. [$^3$H]CE-HDL is a preparation of human HDL containing tritium-labeled cholesteryl ester, and is prepared according to the method of Glenn et al. (Meth. Enzymol., 263, 339-351 (1996)). Test compound is dissolved as a 80 mM stock solution in vehicle (2% ethanol: 98% PEG 400, Sigma Chemical Company, St. Louis, Missouri, USA) and administered either by bolus injection or by continuous infusion. Two minutes after the [$^3$H]CE-HDL dose is administered, animals are to receive 0.1 ml of the test solution injected into the jugular vein. Control animals are to receive 0.1 ml of the intravenous vehicle solution without test compound. After 5 minutes, the first blood samples (0.5 ml) are taken from the carotid artery and collected in standard microtainer tubes containing ethylenediamine tetraacetic acid. Saline (0.5 ml) is injected to flush the catheter and replace blood volume. Subsequent blood samples are to be taken at two hours and four hours by the same method. Blood samples are mixed well and kept on ice until the completion of the experiment. Plasma is obtained by centrifugation of the blood samples at 4° C. The plasma (50 µl) is treated with 5 µl of precipitating reagent (dextran sulfate, 10 g/l; 0.5 M magnesium chloride) to remove VLDL/LDL. After centrifugation, the resulting supernatant (25 µl) containing the HDL will be analyzed for radioactivity using a liquid scintillation counter.

The percentage [$^3$H]CE transferred from HDL to LDL and VLDL (% transfer) will be calculated based on the total radioactivity in equivalent plasma samples before precipitation. Typically, the amount of transfer from HDL to LDL and VLDL in control animals will be 20% to 35% after 4 hours.

Alternatively, conscious, non-anesthetized animals can receive an oral gavage dose of test compound as a suspension in 0.1% methyl cellulose in water. At a time determined for each compound at which plasma levels of the test substance reach their peak ($C_{max}$) after oral dosing, the animals are to be anesthetized with pentobarbital and then dosed with 0.2 ml of a solution containing [$^3$H]CE-HDL into the jugular vein as described above. Control animals are to receive 0.25 ml of the vehicle solution without test compound by oral gavage. After 4 hours, the animals are to be sacrificed, blood samples are collected, and the percentage [$^3$H]CE transferred from HDL to LDL and VLDL (% transfer) is assayed as described above.

Alternatively, inhibition of CETP activity by a test compound can be determined by administering the compound to mice that have been selected for expression of human CETP (hCETP) by transgenic manipulation (hCETP mice). Test compounds can be administered by intravenous injection, or oral gavage and the amount of transfer of tritium-labeled cholesteryl ester ([$^3$H]CE) from HDL to VLDL and LDL particles is determined, and compared to the amount of transfer observed in control animals. C57B1/6 mice that are homozygous for the hCETP gene are to be maintained on a high fat chow diet, such as TD 88051, as described by Nishina et al. (J Lipid Res., 31, 859-869 (1990)) for at least two weeks prior to the study. Mice are to receive an oral gavage dose of test compound as a suspension in 0.1% methyl cellulose in water or an intravenous bolus injection of test compound in 10% ethanol and 90% polyethylene glycol. Control animals are to receive the vehicle solution without test compound by oral gavage or by an intravenous bolus injection. At the start of the experiment all animals will receive 0.05 ml of a solution containing [$^3$H]CE-HDL into the tail vein. [$^3$H]CE-HDL will be a preparation of human HDL containing tritium-labeled cholesteryl ester, and is prepared according to the method of Glenn et al. (Meth. Enzymol., 263, 339-351 (1996)). After 30 minutes, the animals are exsanguinated and blood collected in standard microtainer tubes containing ethylenediamine tetraacetic acid. Blood samples are mixed well and kept on ice until the completion of the experiment. Plasma will be obtained by centrifugation of the blood samples at 4°C. The plasma is separated and analyzed by gel filtration chromatography and the relative proportion of [$^3$H]CE in the VLDL, LDL and HDL regions will be determined.

The percentage [$^3$H]CE transferred from HDL to LDL and VLDL (% transfer) will be calculated based on the total radioactivity in equivalent plasma samples before precipitation. Typically, the amount of transfer from HDL to LDL and VLDL in control animals will be 20% to 35% after 30 min.

## Intestinal Cholesterol Absorption Assay

A variety of compounds are shown to inhibit cholesterol absorption from the intestinal tract. These compounds lower serum cholesterol levels by reducing intestinal absorption of cholesterol from both exogenous sources (dietary cholesterol) and endogenous cholesterol (secreted by the gall bladder into the intestinal tract).

In hamsters the use of a dual-isotope plasma ratio method to measure intestinal cholesterol absorption has been refined and evaluated as described by Turley et al. (J. Lipid Res. 35, 329-339 (1994), herein incorporated by reference).

Male hamsters weighing 80-100 g are to be given food and water ad libitum in a room with 12 hour alternating periods of light and dark. Four hours into the light period, each hamster is administered first an intravenous dose of 2.5 μCi of [1,2-$^3$H]cholesterol suspended in Intralipid (20%) and then an oral dose of [4-$^{14}$C]cholesterol in an oil of medium chain triglycerides (MCT). The i.v. dose is given by injecting a 0.4 ml volume of the Intralipid mixture into the distal femoral vein. The oral dose is given by gavaging a 0.6 ml volume of the MCT oil mixture introduced intragastrically via a polyethylene tube. After 72 hours the hamsters are bled and the amount of $^3$H and $^{14}$C in the plasma and in the original amount of label administered are determined by liquid scintillation spectrometry. The cholesterol absorption will be calculated based on the following equation:

$$\text{Percent cholesterol absorbed} = \frac{\text{\% of oral dose per ml of 72 hour plasma sample}}{\text{\% of i.v. dose per ml of 72 hour plasma sample}} \times 100$$

**Microsomal triglyceride transfer protein (MTP) assay:**

MTP can be purified from liver tissue or cultured cells (e.g. HepG2 cells) using standard methods as described by Ohringer et al. (Acta Crystallogr. D52, 224-225 (1996)).

Subsequent analysis of MTP activity can be performed as described by Jamil et al. (Proc. Natl. Acad. Sci. 93, 11991-11995 (1996).

The basis of this assay is to measure the transfer of labeled triglycerides from a population of donor vesicles to a population of acceptor vesicles in the presence of MTP. Inhibitors of MTP can be evaluated by adding them to the mixture prior to the introduction of MTP. Donor vesicles are prepared by sonication of an aqueous mixture of egg phospholipids, cardiolipin, $^3$H-labeled phospholipid and $^{14}$C-labeled triglycerides. Acceptor vesicles are prepared by sonication of an aqueous mixture of egg phospholipids. The vesicle solutions are mixed together, with or without added MTP inhibitors, and MTP is added to initiate the transfer reaction. The assay is terminated after 60 minutes by addition of 0.5 ml of DE-52 cellulose followed by centrifugation to pellet the donor molecules. The amount of $^3$H and $^{14}$C in the pellet and in the original amount of label in the mixture are determined by liquid scintillation spectrometry. The lipid transfer rate will be calculated based on first order kinetics using the expression:

$$[S] = [S]_0 \, e^{-kt}$$

where $[S]_0$ and [S] are the fractions of $^{14}$C label in the donor membrane pellet at times 0 and t, respectively, and the term k is the fraction of label transferred per unit time.

**Plasma Lipids Assay in Rabbits**

Plasma lipids can be assayed using standard methods as reported by J.R. Schuh et al., J. Clin. Invest., 91, 1453-1458 (1993)). Groups of male, New Zealand white rabbits are placed on a standard diet (100g/day) supplemented with 0.3% cholesterol and 2% corn oil (Zeigler Bothers, Inc., Gardners, PA). Water is available ad lib. Groups of control and treated animals are killed after 1 and 3 months of treatment. Tissues are removed for characterization of atherosclerotic lesions. Blood samples are to be taken for determination of plasma lipid concentrations.

**Plasma Lipids**

Plasma for lipid analysis is to be obtained by withdrawing blood from the ear vein into EDTA-containing tubes (Vacutainer; Becton Dickenson & Co., Rutherford, NJ), followed by centrifugal separation of the cells. Total cholesterol was determined enzymatically, using the cholesterol oxidase reaction (C.A. Allain et al., Clin. Chem., 20, 470-475 (1974). HDL cholesterol was also measured enzymatically, after selective precipitation of LDL and VLDL by dextran sulfate with magnesium (G.R. Warnick et al., Clin. Chem., 28, 1379-1388 (1982)). Plasma triglyceride levels will be determined by measuring the amount of glycerol released by lipoprotein lipase through an enzyme-linked assay (G. Bucolo et al., Clin. Chem., 19, 476-482 (1973)).

**Atherosclerosis**

Animals are to be killed by pentobarbital injection. Thoracic aortas are rapidly removed, immersion fixed in 10% neutral buffered formalin, and stained with oil red O (0.3%). After a single longitudinal incision along the wall

opposite the arterial ostia, the vessels are pinned open for evaluation of the plaque area. The percent plaque coverage is determined from the values for the total area examined and the stained area, by threshold analysis using a true color image analyzer (Videometric 150; American Innovision, Incl, San Diego, CA) interfaced to a color camera (Toshiba 3CCD) mounted on a dissecting microscope. Tissue cholesterol will be measured enzymatically as described, after extraction with a chloroform/methanol mixture (2:1) according to the method of Folch et al. (J. Biol. Chem., 226, 497-509 (1957)).

**In Vitro Vascular Response**

The abdominal aortas are rapidly excised, after injection of sodium pentobarbital, and placed in oxygenated Krebs-bicarbonate buffer. After removal of perivascular tissue, 3-mm ring segments are cut, placed in a 37°C muscle bath containing Krebs-bicarbonate solution, and suspended between two stainless steel wires, one of which is attached to a force transducer (Grass Instrument Co., Quincy, MA). Force changes in response to angiotensin II added to the bath will be recorded on a chart recorder.

**Renal Hypertensive Rat Model**

rison purposes, a combination therapy of an antihypertensive agent and an ileal bile acid transport inhibitor may be evaluated for blood pressure lowering activity in the renal-artery ligated hypertensive rat, a model of high renin hypertension. In this model, six days after litigation of the left renal artery, both plasma renin activity and blood pressure are elevated significantly (J.L. Cangiano et al, J. Pharmacol. Exp. Ther., 206, 310-313 (1979)). Male Sprague-Dawley rats are instrumented with a radiotelemetry blood pressure transmitter for continuous monitoring of blood pressure. The rats are anesthetized with a mixture of ketamine-HCl (100 mg/kg) and acepromazine maleate (2.2 mg/kg). The abdominal aorta is exposed via a midline ncision. Microvascular clamps are placed on the aorta distal to the renal arteries and the iliac bifurcation. The aorta is punctured with a 22-gauge needle and the tip of a catheter is introduced. The catheter, which is held in place by a ligature in the psoas muscle, is connected to a radiotelemetry blood pressure transmitter (Mini-Mitter Co., Inc., Sunriver, OR). The transmitter is placed in the peritoneal cavity and sutured to abdominal muscle upon closing of the incision. Rats are housed singly above a radiotelemetry receiver and are allowed standard rat cho and water *ad libitum*. At least five days are allowed for recovery from surgery. Mean arterial pressure and heart tare are measured on a data recorder as is appropriate, such as a mini-computer. Data Data are sampled for 10 seconds at 200-500 Hz at 2.5 to 10 min intervals 24 hours per day. After collecting control data for 24 hours, the rats are anesthetized with methohexital (30 mg/kg, i.p.) and supplemented as needed. A midline abdominal incision is made, approximately 2 cm in length to expose the left kidney. The renal artery is separated from the vein near the aorta, with care taken not to tranatize the vein. The artery is completely ligated with sterile 4-O silk. The incision is closed by careful suturing of the muscle layer and skin. Six days later, when MAP is typically elevated by 50-70 mmHg, an antihypertensive agent or a combination with one or more cardiovascular therapeutic agents are administerd by gavage each day for about 8 weeks. Single drug dosing is carried out using 20 and 200 mg/kg/day of the antihypertensive agent (for example, eplerenone) and 1, 3, 10, 30, and 100 mg/kg/day of the other cardiovascular therapeutic agent. Drug mixtures are obtained by administering a combination of a dose of 1, 3, 10, 30, or 100 mg/kg/day of the other cardiovascular therapeutic agent with a dose of either 20 or 200 mg/kg/day of the antihypertensive agent. Blood pressure lowering is monitored by the radiotelemetry system and responses with the compounds are compared to a response obtained in vehicle-treated animals. Plasma and urinary sodium and potassium levels are monitored as a measure of the effectiveness of the aldosterone blockade. Urine samples are collected overnight using metabolic cages to isolate the samples. Plasma samples are obtained by venous catheterization. Sodium and potassium are measured by flame photometry. Cardic fibrosis is determined by histological and chemical measurements of the excised hearts following perfusion fixation. Left and right ventricles are weighed, embedded, and sectioned. Subsequently, sections are stained with picrosirius red and the red staining collagen areas are quantitated by computerized image analysis. The apex of th heart is acid digested and the free hydroxyproline measured colorimetrically. It is expected that MAP will be significantly lowered toward normal pressures in the test animals, treated with the combination therapy and that the condition of myocardial fibrosis will be arrested or avoided.

**Effect of an IBAT Inhibitor and an Antihypertensive Agent, Alone and in Combination, on the Treatment of Atherosclerosis**

This comparative study will be a prospective randomized evaluation of the effect of a combination of an IBAT inhibitor or a pharmaceutically acceptable salt thereof and an antihypertensive agent on the progression/regression of coronary and carotid artery disease. The study is used to show that a combination of an IBAT inhibitor or a pharmaceutically acceptable soft thereof and an antihypertensive agent is effective in slowing or arresting the progression or

causing regression of existing coronary artery disease (CAD) as evidenced by changes in coronary angiography or carotid ultrasound in subjects with established disease.

This study will be an angiographic documentation of coronary artery diseasecarried out as a double-blind, placebo-controlled trial of a minimum of about 500 subjects and preferably of about 780 to about 1200 subjects. It is especially preferred to study about 1200 subjects in this study. Subjects will be admitted into the study after satisfying certain entry criteria set forth below.

**Entry criteria:** Subjects accepted for entry into this trial must satisfy certain criteria. Thus the subject must be an adult, either male or female, aged 18-80 years of age in whom coronary angiography is clinically indicated. Subjects will have angiographic presence of a significant focal lesion such as 30% to 50% on subsequent evaluation by quantitative coronary angiography (QCA) in a minimum of one segment (non-PTCA, non-bypassed or non-MI vessel) that is judged not likely to require intervention over the next 3 years. It is required that the segments undergoing analysis have not been interfered with. Since percutaneous transluminal cardiac angioplasty (PTCA) interferes with segments by the insertion of a balloon catheter, non-PTCA segments are required for analysis. It is also required that the segments to be analyzed have not suffered a thrombotic event, such as a myocardial infarct (MI). Thus the requirement for non-MI vessels. Segments that will be analyzed include: left main, proximal, mid and distal left anterior descending, first and second diagonal branch, proximal and distal Left circumflex, first or largest space obtuse marginal, proximal, mid and distal right coronary artery. Subjects will have an ejection fraction of greater than 40% determined by catheterization or radionuclide ventriculography or ECHO cardiogram at the time of the qualifying angiogram or within the previous three months of the acceptance of the qualifying angiogram provided no intervening event such as a thrombotic event or procedure such as PTCA has occurred.

Generally, due to the number of patients and the physical limitations of any one facility, the study will be carried out at multiple sites. At entry into the study, subjects undergo quantitative coronary angiography as well as B-mode carotid artery ultrasonography and assessment of carotid arterial compliance at designated testing centers. This will establish baselines for each subject. Once admitted into the test, subjects are randomized to receive an antihypertensive agent (for example, eplerenone) or a pharmaceutically acceptable salt thereof (the dose is dependent upon the particular antihypertensive agent or salt thereof chosen) and placebo or antihyperlipidemic agent such as an IBAT inhibitor (50 mgs) and placebo or an antihypertensive agent or a pharmaceutically acceptable salt thereof (the dose is dependent upon the particular antihypertensive agent or salt thereof chosen) and IBAT inhibitor (50 mgs). It will be recognized by a skilled person that the free base form or other salt forms of antihypertensive agent or the free base form or other salt forms of the IBAT inhibitor may be used.

Calculation of the dosage amount for these other forms of the IBAT inhibitor and amlodipine besylate is easily accomplished by performing a simple ratio relative to the molecular weights of the species involved. The amount of the antihypertensive agent may be varied as required. The amount of the IBAT inhibitor will be titrated down from 80 mg if it is determined by the physician to be in the best interests of the subject. The subjects are monitored for a one to three year period, generally three years being preferred. B-mode carotid ultrasound assessment of carotid artery atherosclerosis and compliance are performed at regular intervals throughout the study. Generally, six month intervals are suitable. Typically this assessment is performed using B-mode ultrasound equipment. However, a person skilled in the art may use other methods of performing this assessment coronary angiography is performed at the conclusion of the one to three year treatment period. The baseline and post-treatment angiograms and the intervening carotid artery B-mode ultrasonograms are evaluated for new lesions or progression of existing atherosclerotic lesions. Arterial compliance measurements are assessed for changes from baseline and over the 6-month evaluation periods.

The primary objective of this study is to show that the combination of an antihypertensive agent and an IBAT inhibitor reduces the progression of atherosclerotic lesions as measured by quantitative coronary angiography (QCA) in subjects with clinical coronary artery disease. QCA measures the opening in the lumen of the arteries measured.

The primary endpoint of the study is the change in the average mean segment diameter of the coronary artery tree. Thus, the diameter of an arterial segment is measured at various portions along the length of that segment. The average diameter of that segment is then determined. After the average segment diameter of many segments has been determined, the average of all segment averages is determined to arrive at the average mean segment diameter. The mean segment diameter of subjects taking the IBAT inhibitor or a pharmaceutically acceptable salt thereof and the antihypertensive agent or a pharmaceutically acceptable acid addition salt thereof will decline more slowly, will be halted completely, or there will be an increase in the mean segment diameter. These results will represent slowed progression of atherosclerosis, halted progression of atherosclerosis and regression of atherosclerosis, respectively.

The secondary objective of this study is that the combination of an antihypertensive agent and the IBAT inhibitor or a pharmaceutically acceptable salt thereof reduces the rate of progression of atherosclerosis in the carotid arteries as measured by the slope of the maximum intimal-medial thickness measurements averaged over 12 separate wall segments (Mean Max) as a function of time, more than does amlodipine or a pharmaceutically acceptable acid addition salt thereof or IBAT inhibitor or a pharmaceutically acceptable salt thereof alone. The intimal-medial thickness of subjects taking an IBAT inhibitor or a pharmaceutically acceptable salt thereof and amlodipine or a pharmaceutically

acceptable acid addition salt thereof will increase more slowly, will cease to increase or will decrease. These results represent slowed progression of atherosclerosis, hafted progression of atherosclerosis and regression of atherosclerosis, respectively. Further, these results may be used to facilitate dosage determinations.

The utility of the compounds as medical agents in the treatment of angina pectoris in mammals (e.g., humans) Is demonstrated by the activity of the compounds in conventional assays and the clinical protocol described below:

**Effect of IBAT Inhibitor and an Antihypertensive Agent, Alone and in Combination. on the Treatment of Angina**

This study will be a double blind, parallel arm, randomized study to show the effectiveness of an IBAT inhibitor or a pharmaceutically acceptable salt thereof and an antihypertensive agent given in.combination in the treatment of symptomatic angina.

**Entry criteria:** Subjects are males or females between 18 and 80 years of age with a history of typical chest pain associated with one of the following objective evidences of cardiac ischemia: (1) stress test segment elevation of about one millimeter or more from the ECG; (2) positive treadmill stress test; (3) new wall motion abnormality on ultrasound; or (4) coronary angiogram with a significant qualifying stenosis. Generally a stenosis of about 30-50% is considered to be significant

Each subject is evaluated for about ten to thirty-two weeks. At least ten weeks are generally required to complete the study. Sufficient subjects are used in this screen to ensure that about 200 to 800 subjects and preferably about 400 subject are evaluated to complete the study. Subjects are screened for compliance with the entry criteria, set forth below, during a four week run in phase. After the screening criteria are met, subjects are washed out from their current ant-anginal medication and stabilized on a long acting nitrate such as nitroglycerine, isosorbide-5-mononitrate or isosorbide dinitrate. The term "washed out", when used in connection with this screen, means the withdrawal of current anti-anginal medication so that substantially all of the medication is eliminated from the body of the subject A period of eight weeks is preferably allowed for both the wash out period and for the establishment of the subject on stable doses of the nitrate. Subjects having one or two attacks of angina per week while on stable doses of long acting nitrate are generally permitted to skip the wash out phase. After subjects are stabilized on nitrates, the subjects enter the randomization phase provided the subjects continue to have either one or two angina attacks per week. In the randomization phase, the subjects are randomly placed into one of the four arms of the study set forth below. After completing the wash out phase, subjects in compliance with the entry criteria undergo twenty four hour ambulatory electrocardigram (ECG) such as Holter monitoring, exercise stress testing such as a treadmill and evaluation of myocardial perfusion using PET (photon emission tomography) scanning to establish a baseline for each subject. When conducting a stress test, the speed of the treadmill and the gradient of the treadmill can be controlled by a technician. The speed of the treadmill and the angle of the gradient are generally increased during the test. The time intervals between each speed and gradient Increase is generally determined using a modified Bruce Protocol.

After the baseline investigations have been completed, subjects are initiated on one of the following four arms of the study: (1) placebo; (2) IBAT inhibitor (about 1 mg to about 80 mg); (3) an antihypertensive agent (dose is dependent upon the particular antihypertensive agent chosen); or (4) a combination of the above doses of IBAT inhibitor and antihypertensive agent together. It will be recognized by a skilled person that the tee base form or other salt forms of amlodipine besylate or the free base form or other salt forms of the IBAT inhibitor may be used . Calculation of the dosage amount for these other forms of the IBAT inhibitor and amlodipine besylate is easily accomplished by performing a simple ratio relative to the molecular weights of the species involved. The subjects are then monitored for two to twenty four weeks.

After the monitoring period has ended subjects will undergo the following investigations: (1) twenty four hour ambulatory ECG, such as Holler monitoring*, (2) exercise stress testing (e.g. treadmill using the modified Bruce Protocol); and (3) evaluation of myocardial perfusion using PET scanning. Patients keep a diary of painful ischemic events and nitroglycerine consumption. It is generally desirable to have an accurate record of the number of anginal attacks suffered by the patient during ' the duration of the test Since a patient generally takes nitroglycerin to ease the pain of an anginal attack, the number of times that the patient administers nitroglycerine provides a reasonably accurate record of the number of anginal attacks.

To demonstrate the effectiveness and dosage of drug combination, the person conducting the test will evaluate the subject using the tests described. Successful treatment Wit yield fewer instances of ischemic events as detected by ECG, will allow the subject to exercise longer or at a higher intensity level on the treadmill, or to exercise without pain on the treadmill, or will yield better perfusion or fewer perfusion defects an ultrasound.

The utility of the compounds as medical agents in the treatment of hypertension and hyperlipidemia in mammals (e.g., humans) suffering from a combination of hypertension and hyperlipidemia is demonstrated by the activity of the compounds in conventional assays and the clinical protocol described below,

**Effect of an IBAT Inhibitor and an Antihypertensive Agent Alone and In Combination on the Treatment of Subjects Having Both Hypertension and Hyperlipidemia**

This study will be a double blind, parallel arm, randomized study to show the effectiveness of an IBAT inhibitor or a pharmaceutically acceptable salt thereof and an antihypertensive agent given in combination in controlling both hypertension and hyperlipidemia in subjects who have mild, moderate, or severe hypertension and hyperlipidiemia

Each subject is evaluated for 10 to 20 weeks and preferably for 14 weeks. Sufficient subjects are used in this screen to ensure that about 400 to 800 subjects are evaluated to complete the study.

Entry criteria: Subjects are male or female adults between 18 and 80 years of age having both hyperlipidemia and hypertension. The presence of hyperlipidemia is evidenced by evaluation of the low density lipoprotein (LDL) level of the subject relative to certain positive risk factors. If the subject has no coronary heart disease (CHD) and has less than two positive risk factors, then the subject is considered to have hyperlipidemia which requires drug therapy if the LDL of the subject is greater than or equal to 190. If the subject has no CHD and has two or more positive risk factors, then the subject is considered to have hyperlipidemia which requires drug therapy if the LDL of the subject is greater than or equal to 160. If the subject has CHID, then the subject is considered to have hyperlipidemia if the LDL of the subject is greater than or equal to 130.

Positive risk factors include (1) male over 45, (2) female over 55 wherein the female is not undergoing hormone replacement therapy (HIRT), (3) family history of premature cardiovascular disease, (4) the subject is a current smoker, (5) the subject has diabetes, (6) an HDL of less than 45, and (7) the subject has hypertension. An HDL of greater than 60 is considered a negative risk factor and will offset one of the above mentioned positive risk factors. The presence of hypertension is evidenced by a sitting diastolic blood pressure (BP) of greater than 90 or sitting systolic BP of greater than 140. All blood pressures are generally determined as the average of three measurements taken five minutes apart. Subjects are screened for compliance with the entry criteria set forth above. After all screening criteria are met, subjects are washed out from their current antihypertensive and lipid lowering medication and are placed on the NCEP ATP 11 Step 1 diet. The NCEP ATP 11 (adult treatment panel, 2nd revision) Step I diet sets forth the amount of saturated and unsaturated fat which can be consumed as a proportion of the total caloric intake. The term "washed out' where used in connection with this screen, means the withdrawal of current antihypertensive and lipid lowering medication so that substantially all of the medication is eliminated from the body of the subject. Newly diagnosed subjects generally remain untreated until the test begins. These subjects are also placed on the NCEP Step I diet. After the four week wash out and diet stabilization period, subjects undergo the following baseline investigations: (1) blood pressure and (2) fasting lipid screen. The fasting lipid screen determines baseline lipid levels in the fasting state of a subject Generally, the subject abstains from food for twelve hours, at which time lipid levels are measured. After the baseline investigations are performed subjects are started on one of the following: (1) a fixed dose of an antihypertensive agent, dose dependent upon the particular antihypertensive agent chosen; (2) a fixed dose of an IBAT inhibitor, generally about 1 to 80mg; or (3) a combination of the above doses of the IBAT inhibitor and the antihypertensive agent together. It will be recognized by a skilled person that the free base form or other salt forms of amlodipine besylate or the free base form or other salt forms of the IBAT inhibitor may be used. Calculation of the dosage amount for these other forms of the IBAT inhibitor and amlodipine besylate is easily accomplished by performing a simple ratio relative to the molecular weights of the species involved. Subjects remain on these doses for a minimum of six weeks, and generally for no more than eight weeks. The subjects return to the testing center at the conclusion of the six to eight weeks so that the baseline evaluations can be repeated. The blood pressure of the subject at the conclusion of the study is compared with the blood pressure of the subject upon entry. The lipid screen measures the total cholesterol, LDL-cholesterol, HDL-cholesterol, triglycerides, apoB, VLDL (very low density lipoprotein) and other components of the lipid profile of the subject. Improvements in the values obtained after treatment relative to pretreatment values indicate the utility of the drug combination. The utility of the compounds as medical agents in the management of cardiac risk in mammals (e.g., humans) at risk for an adverse cardiac event is demonstrated by the activity of the compounds in conventional assays and the clinical protocol described below.

**Effects of an IBAT Inhibitor and an Antihypertensive Agent, Alone and in Combination, on Subjects at Risk of Future Cardiovascular Events**

This study will be a double blind, parallel arm, randomized study to show the effectiveness of an IBAT inhibitor or a pharmaceutically acceptable salt thereof and antihypertensive agent given in combination in reducing the overall calculated risk of future events in subjects who are at risk for having future cardiovascular events. This risk is calculated by using the Framingham Risk Equation. A subject is considered to be at risk of having a future cardiovascular event if that subject is more than one standard deviation above the mean as calculated by the Framingham Risk Equation. The study is used to evaluate the efficacy of a fixed combination of the IBAT inhibitor or a pharmaceutically acceptable salt thereof and the antihypertensive agent in controlling cardiovascular risk by controlling both hypertension and hyperlipidemia in patients who have both mild to moderate hypertension and hyperlipidemia.

Each subject is evaluated for 10 to 20 weeks and preferably for 14 weeks. Sufficient subjects are recruited to ensure that about 400 to 800 subjects are evaluated to complete the study.

**Entry criteria:** Subjects included in the study are male or female adult subjects between 18 and 80 years of age with a baseline five year risk which risk is above the median for the subject's age and sex, as defined by the Framingham Heart Study, which is an ongoing prospective study of adult men and women showing that certain risk factors can be used to predict the development of coronary heart disease. The age, sex, systolic and diastolic blood pressure, smoking habit, presence or absence of carbohydrate intolerance, presence or absence of left ventricular hypertrophy, serum cholesterol and high density lipoprotein (HDL) of more than one standard deviation above the norm for the Framingham Population are all evaluated in determining whether a patent is at risk for adverse cardiac event. The values for the risk factors are inserted into the Framingham Risk equation and calculated to determine whether a subject is at risk for a future cardiovascular event. Subjects are screened for compliance with the entry criteria set forth above. After all screening criteria are met, patients are washed out from their current antihypertensive and lipid lowering medication and any other medication which will impact the results of the screen. The patients are then placed on the NCEP ATP 11 Step I diet, as described above. Newly diagnosed subjects generally remain untreated until the test begins- These subjects are also placed on the NCEP ATP 11 Step 1 diet. After the four week wash out and diet stabilization period, subjects undergo the following baseline investigations: (1) blood pressure; (2) fasting; (3) DPW screen; (4) glucose tolerance test; (5) ECG; and (6) cardiac ultrasound. These tests are carried out using standard procedures well known to persons skilled in the art The ECG and the cardiac ultrasound are generally used to measure the presence or absence of left ventricular hypertrophy.

After the baseline investigations are performed patents will be started on one of the following: (1) a fixed dose of an antihypertensive agent, dose dependent upon the particular antihypertensive agent chosen; (2) a fixed dose of an IBAT inhibitor (about 1 to 80mg); or (3) the combination of the above doses of the IBAT inhibitor and an antihypertensive agent. It will be recognized by a skilled person that the free base form or other salt forms of amlodipine besylate or the free base form or other salt forms of the IBAT inhibitor may be used,

Calculation of the dosage amount for these other forms of the IBAT inhibitor and amlodipine besylate is easily accomplished by performing a simple ratio relative to the molecular weights of the species involved. Patients are kept on these doses and are asked to return in six to eight weeks so that the baseline evaluations can be repeated. At this time the new values are entered into the Framingham Risk equation to determine whether the subject has a lower, greater or no change in the risk of future cardiovascular event

The above assays demonstrating the effectiveness of amlodipine or pharmaceutically acceptable acid addition salts thereof and an IBAT inhibitor or pharmaceutically acceptable salts thereof in the treatment of angina pectoris, atherosclerosis, hypertension and hyperlipidemia together, and the management of cardiac risk, also provide a means whereby the activities of the compounds can be compared between themselves and with the activities of other known compounds. The results of these comparisons are useful for determining dosage levels in mammals, including humans, for the treatment of such diseases. The following dosage amounts and other dosage amounts set forth elsewhere in this specification and in the appendant claims are for an average human subject having a weight of about 65 kg to about 70 kg. The skilled practitioner will readily be able to determine the dosage amount required for a subject whose weight falls outside the 65 kg to 70 kg range, based upon the medical history of the subject and the presence of diseases, e.g., diabetes, in the subject. All doses set forth herein, and in the appendant claims, are daily doses.

By way of general example, the below-listed antihypertensive agent may be administered in the following daily dosage amounts:

diltiazem, generally about 120 mg to about 480 mg;
verapamil, generally about 20 mg to about 48 mg;
felodipine, generally about 2.5 mg to about 40 mg;
isradipine, generally about 2.5 mg to about 40 mg;
lacidipine, generally about 1 mg to about 6 mg;
nicardipine, generally about 32 mg to about 120 mg;
nifedipine, generally about 10 mg to about 120 mg;
nimodipine, generally about 120 mg to about 480 mg;
nisoldipine, generally about 5 mg to about 80 mg;
nitrendipine, generally about 5 mg to about 20 mg;
benazepril, generally about 10 mg to about 80 mg;
captopril, generally about 50 mg to about 150 mg;
enalapril, generally about 5 mg to about 40 mg;
fosinopril, generally about 10 mg to about 80 mg;
lisinopril, generally about 10 mg to about 80 mg;

quinapril, generally about 10 mg to about 80 mg;
losartan, generally about 25 mg to about 100 mg;
valsartan, generally about 40 mg to about 640 mg;
doxazosin, generally about 0.5 mg to about 16 mg;
prazosin, generally about 1 mg to about 40 mg;
trimazosin, generally about 1 mg to about 20 mg;
arniloride, generally about 5 mg to about 20 mg; and
eplerenone, generally about 10 to about 150 mg.

It will be recognized by those skilled in the art that dosages for the above antihypertensive compounds must be individualized to each specific subject. This individualization will depend upon the medical history of the subject and whether the subject is concurrently taking other medications which may or may not interfere or have an adverse effect in combination with the above antihypertensives. Individualization is then achieved by beginning with a low dose of the compound and titrating the amount up until the desired therapeutic effect is achieved. In general, the IBAT inhibitor is generally administered in a dosage of about 0.1 mg/day to about 500 mg/day. Preferably, the IBAT inhibitor is administered in a dosage of about 1 mg/day to about 100 mg/day.

Since the present invention relates to the treatment of diseases and conditions with a combination of active ingredients which may be administered separately, the invention also relates to combining separate pharmaceutical compositions in kit form. The kit includes two separate pharmaceutical compositions.

The kit includes container means for containing the separate compositions such as a divided bottle or a divided foil packet however, the separate compositions may also be contained within a single, undivided container. Typically the kit includes directions for the administration of the separate components. The kit form is particularly advantageous when the separate components are preferably administered in different dosage forms (e.g., oral and parenteral), are administered at different dosage intervals, or when titration of the individual components of the combination is desired by the prescribing physician.

The examples herein can be performed by substituting the generically or specifically described therapeutic compounds or inert ingredients for those used in the preceding examples.

The invention being thus described, it is apparent that the same can be varied in many ways. Such variations are not to be regarded as a departure from the scope of the present invention, and all such modifications and equivalents as would be obvious to one skilled in the art are intended to be included within the scope of the following claims.

**Claims**

1. A therapeutic combination comprising a first amount of an ileal bile acid transport inhibiting compound and a second amount of a microsomal triglyceride transfer protein inhibiting compound wherein the first amount and the second amount together comprise an anti-hyperlipidemic condition effective amount, an anti-atherosclerotic condition effective amount, or an anti-hypercholesterolemic condition effective amount of the compounds.

2. The therapeutic combination of claim 1 wherein the ileal bile acid transport inhibiting compound has one of the following structures:

(1)

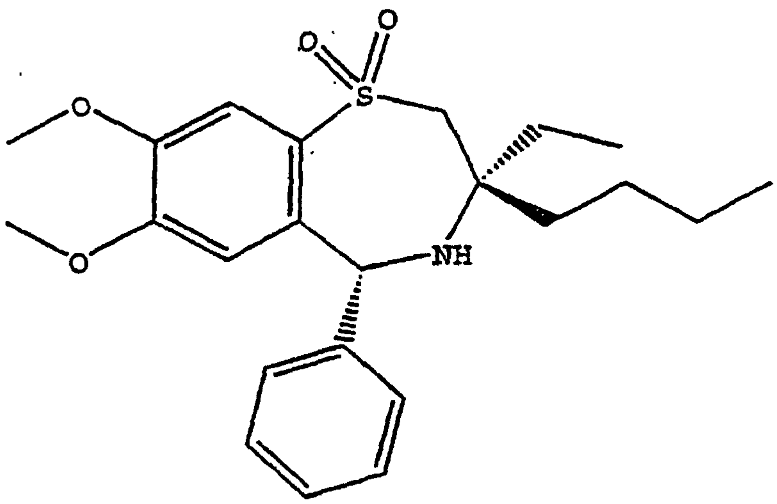

B-2

or an enantiomer or racemate thereof;

(2)

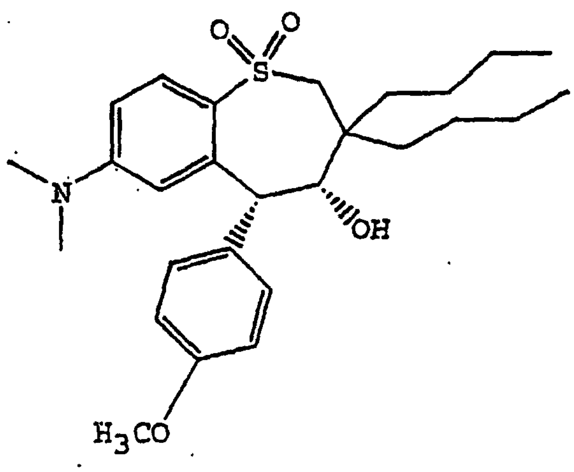

B-12

or an enantiomer or racemate thereof;

(3)

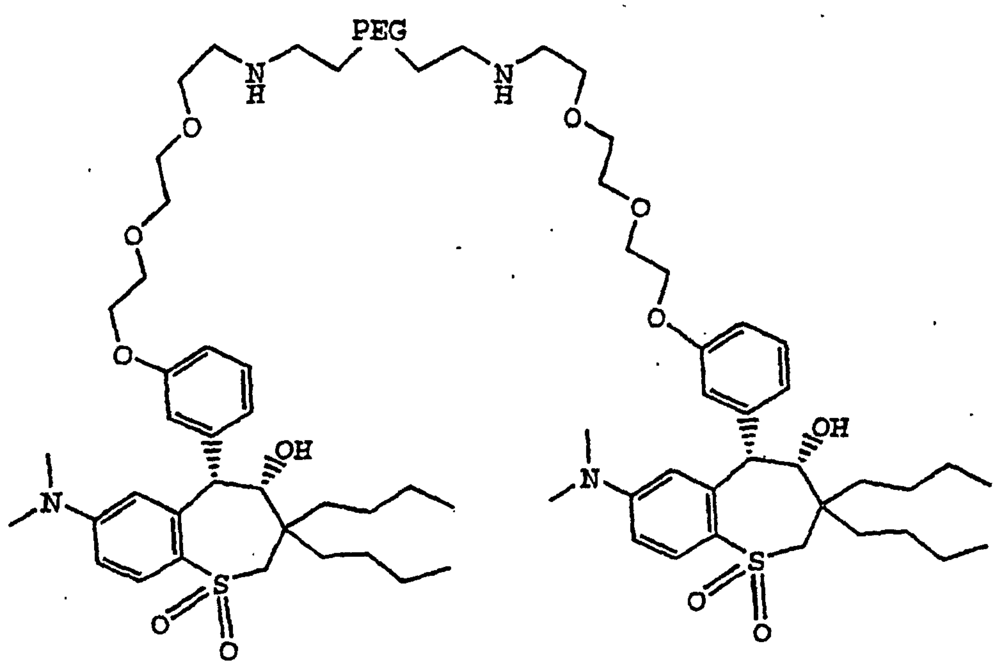

or an enantiomer or racemate thereof, wherein PEG is an about 3000 to about 4000 molecular weight polyethylene glycol polymer chain.

(4)

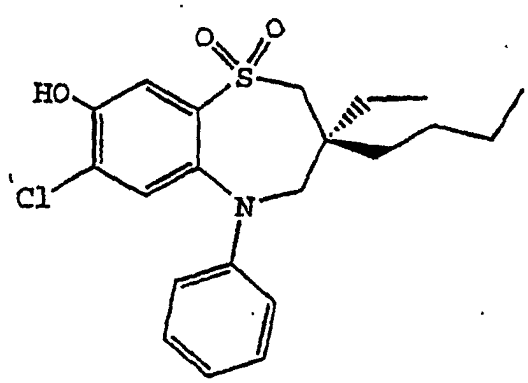

B-7

or an enantiomer or racemate thereof.

3. Use of a first amount of an ileal bile acid transport inhibiting compound and a second amount of a microsomal triglyceride transfer protein inhibiting compound for the manufacture of a medicament for the prophylaxis or treatment of a hyperlipidemic condition or disorder in a mammal, wherein the first amount and the second amount together comprise an anti-hyperlipidemic condition effecitive amount, an anti-atherosclerotic condition effective amount, or an anti-hyperchloesterolemic condition effective amount of the compounds.

4. A kit for achieving a therapeutic effect in a mammal comprising an amount of an ileal bile acid transport inhibiting compound in a first unit dosage form; an amount
of a microsomal triglyceride transfer protein inhibiting compound

in a second unit dosage form; and container means for containing said first and second unit dosage forms.

**5.** A therapeutic combination comprising a first amount of an ileal bile acid transport (IBAT) inhibiting compound and a second amount of a microsomal triglyceride transfer protein (MTP) inhibiting compound,

wherein said first amount and said second amount together comprise an anti-hyperlipidemic condition effective amount, an anti-atherosclerotic condition effective amount, or an anti-hypercholesteroemic condition effective amount of said (IBAT) inhibiting and said MTP inhibiting compounds, and

wherein said MTP inhibiting compound is selected from the group consisting of M-1, M-2, M-3, M-4, M-5, M-6, M-7, M-8, and M-9 represented by:

M-1

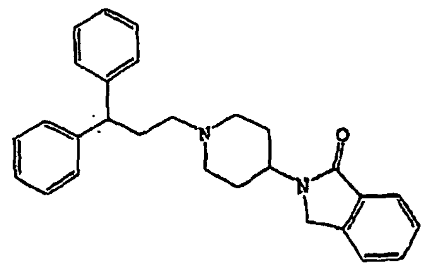

M-2

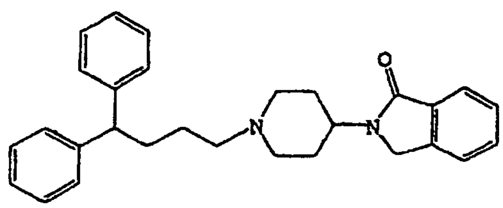

M-3

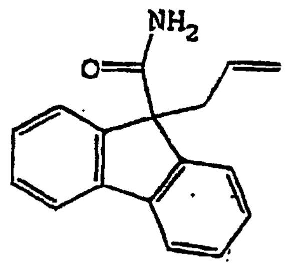

M-4

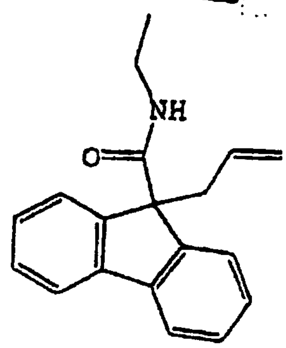

M-5

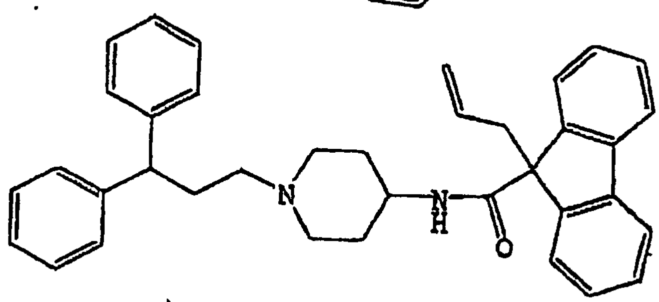

M-6

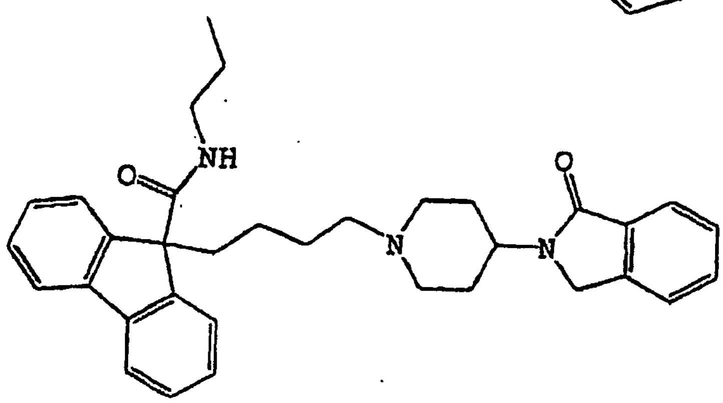

M-7

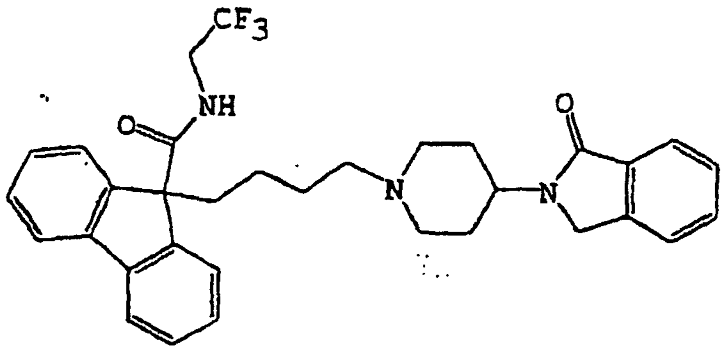

M-8

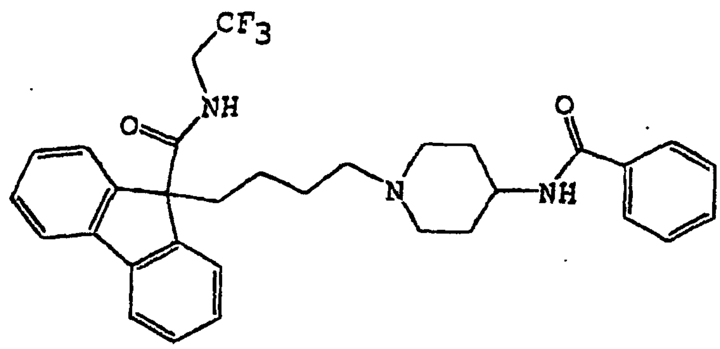

and

M-9

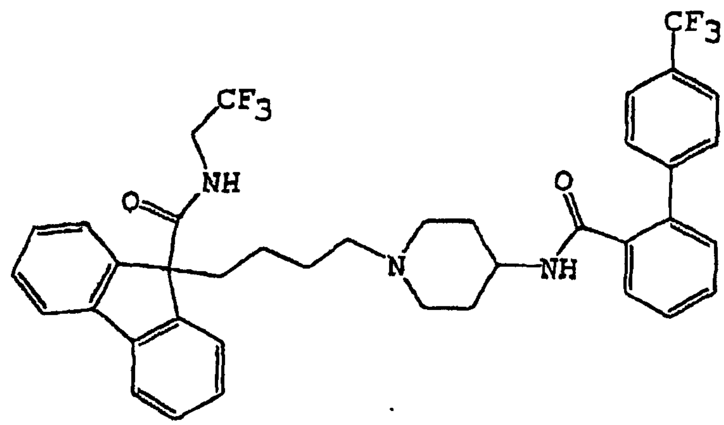

and enantiomers, racemates, tautomers and salts thereof.

**6.** The therapeutic combination of claim 5 wherein said IBAT inhibiting compound is represented by formula B-5:

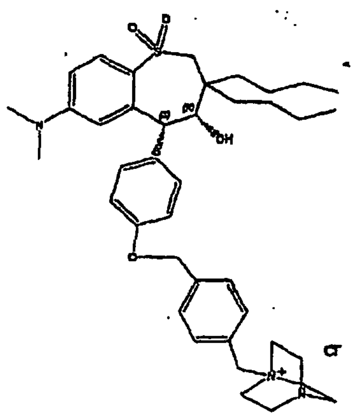

(B-5)

or enantiomers or racemates thereof

**7.** The therapeutic combination of claim 6, wherein said MTP inhibiting compound is represented by formula M-9:

M-9

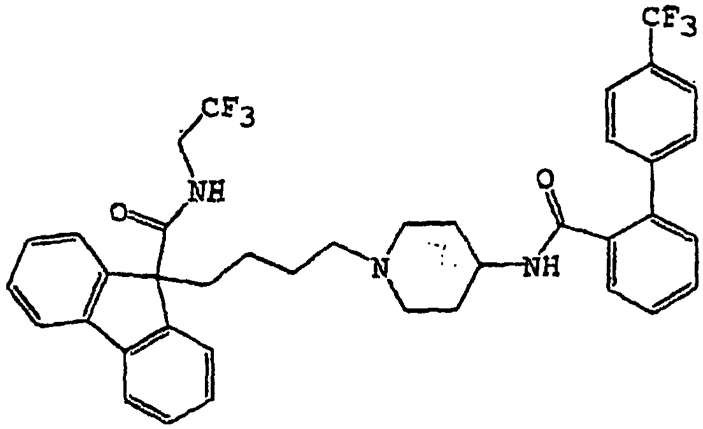

## Patentansprüche

1. Therapeutische Kombination, umfassend eine erste Menge einer den ilealen Gallensäuretransport hemmenden Verbindung und eine zweite Menge einer das mikrosomale Triglycerid-Transferprotein hemmenden Verbindung, worin die erste Menge und die zweite Menge zusammen eine gegen einen hyperlipidämischen Zustand wirksame Menge, eine gegen einen aterosklerotischen Zustand wirksame Menge oder eine gegen einen hypercholesterinämischen Zustand wirksame Menge der Verbindungen umfassen.

2. Therapeutische Kombination nach Anspruch 1, worin die den ilealen Gallensäuretransport hemmende Verbindung eine der folgenden Strukturen besitzt:

(1)

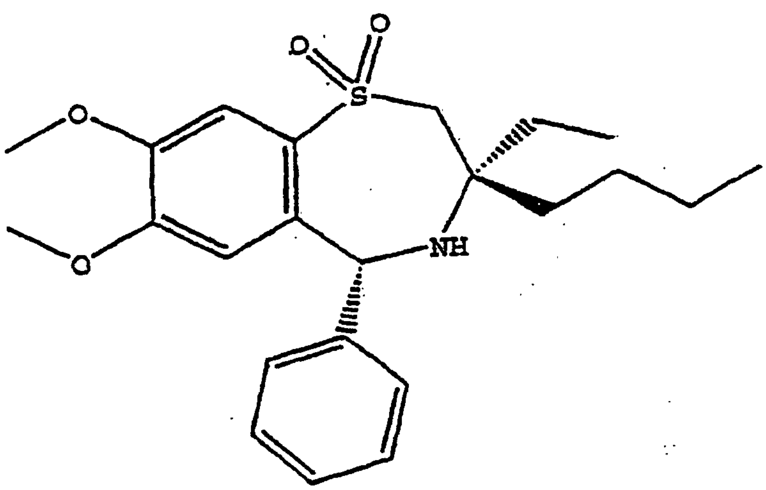

B-2

oder ein Enantiomer oder Racemat davon;

(2)

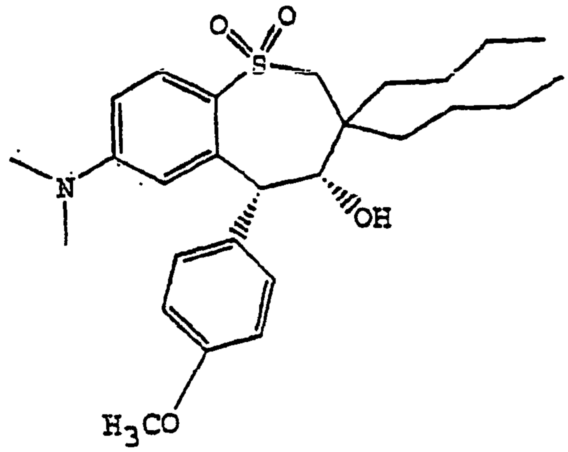

B-12

oder ein Enantiomer oder Racemat davon;

(3)

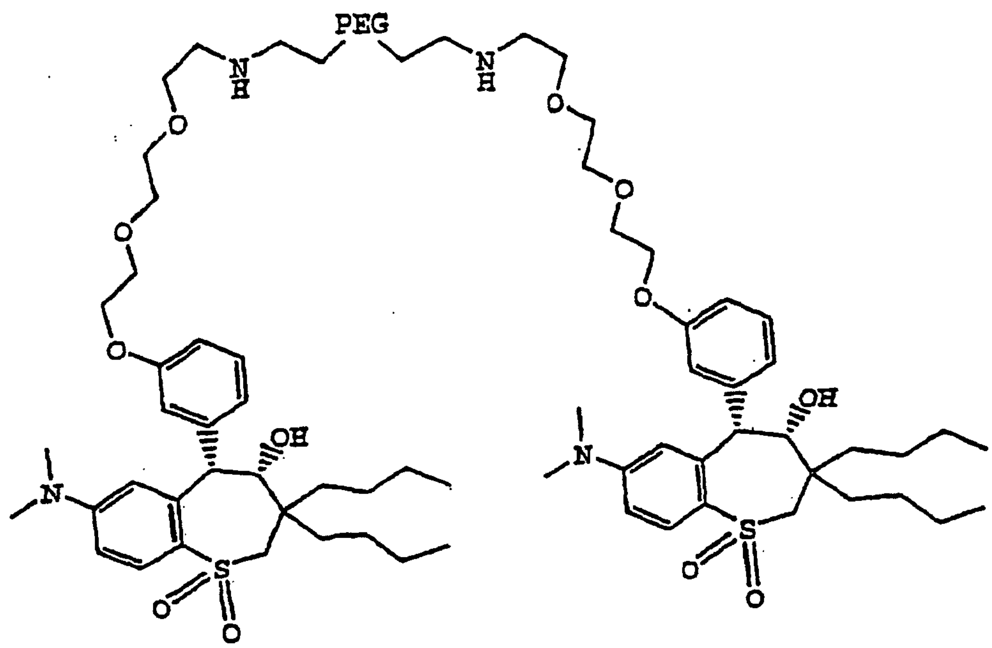

oder ein Enantiomer oder Racemat davon, wobei PEG eine Polyethylenglykolpolymerkette mit einem Molekulargewicht von etwa 3000 bis etwa 4000 ist;

(4)

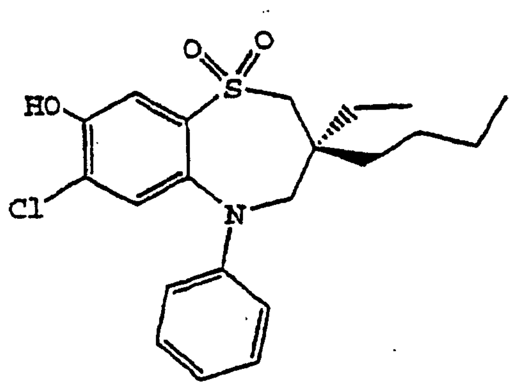

B-7

oder ein Enantiomer oder Racemat davon.

3. Verwendung einer ersten Menge einer den ilealen Gallensäuretransport hemmenden Verbindung und einer zweiten Menge eines das mikrosomale Triglycerid-Transferprotein hemmenden Verbindung zur Herstellung eines Medikaments zur Prophylaxe oder Behandlung eines hyperlipidämischen Zustands oder einer hyperlipidämischen Erkrankung an einem Säugetier, wobei die erste Menge und die zweite Menge zusammen eine gegen einen hyperlipidämischen Zustand wirksame Menge, eine gegen einen aterosklerotischen Zustand wirksame Menge, oder eine gegen einen hypercholesterinämischen Zustand wirksame Menge der Verbindungen umfassen.

4. Kit zum Erhalt eines therapeutischen Effekts an einem Säugetier umfassend eine Menge einer den ilealen Gallensäuretransport hemmenden Verbindung in einer ersten Dosierungseinheitsform; eine Menge einer das mikrosomale Triglycerid-Transferprotein hemmenden Verbindung in einer zweiten Dosierungseinheitsform; und ein Behältnis zur Aufnahme der genannten ersten und zweiten Dosierungseinheitsformen.

5. Therapeutische Kombination umfassend eine erste Menge einer den ilealen Gallensäuretransport (IBAT) hemmenden Verbindung und eine zweite Menge einer das mikrosomale Triglycerid-Transferprotein (MTP) hemmenden Verbindung,
wobei die genannte erste Menge und die genannte zweite Menge zusammen eine gegen einen hyperlipidämischen Zustand wirksame Menge, eine gegen einen aterosklerotischen Zustand wirksame Menge oder eine gegen einen hypercholesterinämischen Zustand wirksame Menge der genannten IBAT-hemmenden und der genannten MTP-hemmenden Verbindungen umfassen und
worin die genannte MTP-hemmende Verbindung ausgewählt ist aus der Gruppe bestehend aus M-1, M-2, M-3, M-4, M-5, M-6, M-7, M-8 und M-9, dargestellt durch:

M-1

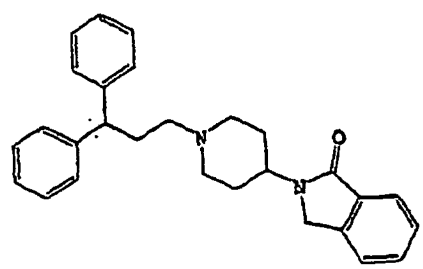

M-2

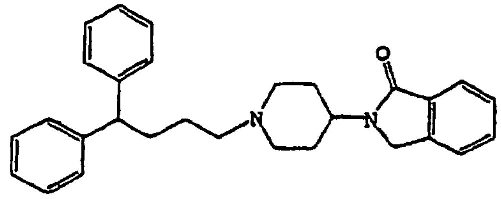

M-3

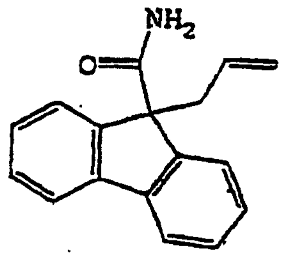

M-4

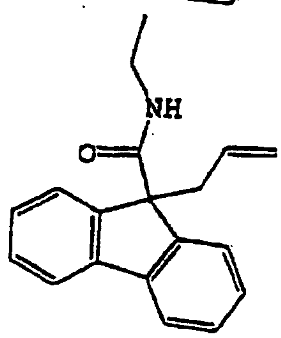

M-5

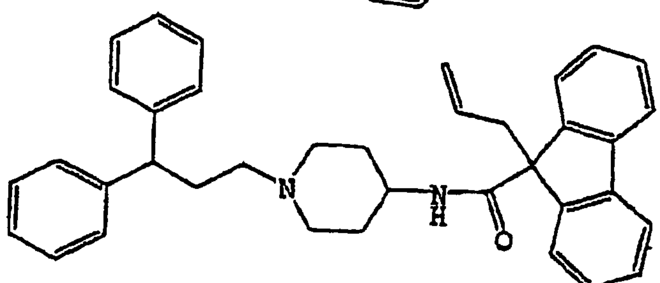

M-6

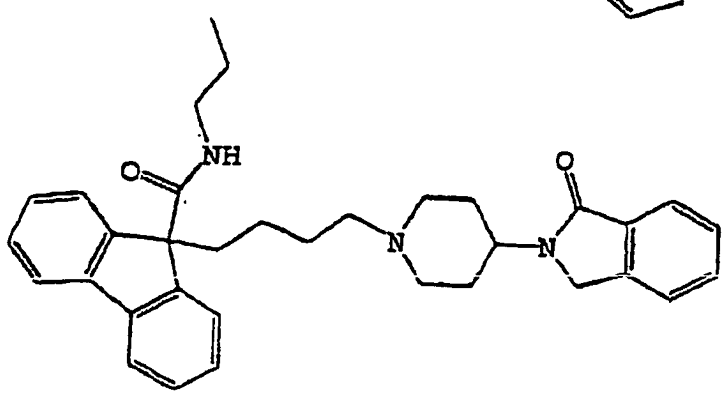

M-7

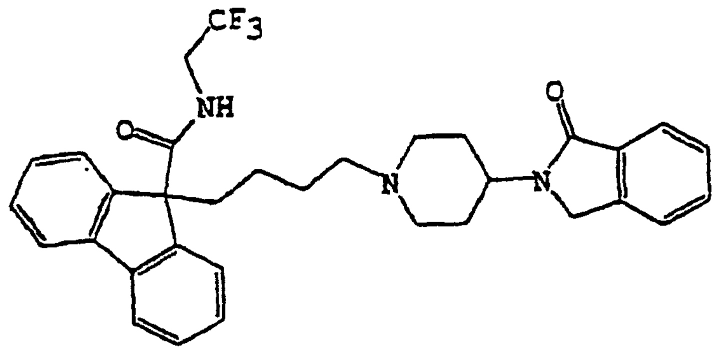

M-8

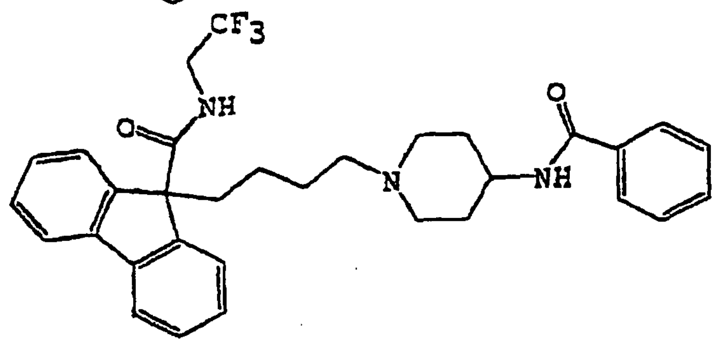

und

M-9

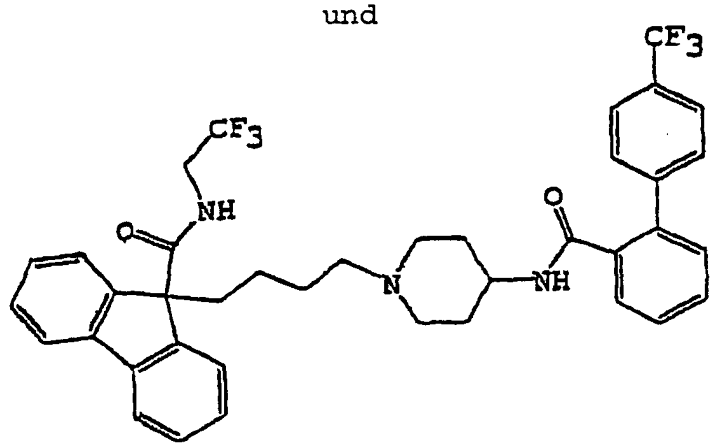

sowie Enantiomeren, Racematen, Tautomeren und Salzen davon.

6. Therapeutische Kombination nach Anspruch 5, worin die genannte IBAT-hemmende Verbindung durch Formel B-5:

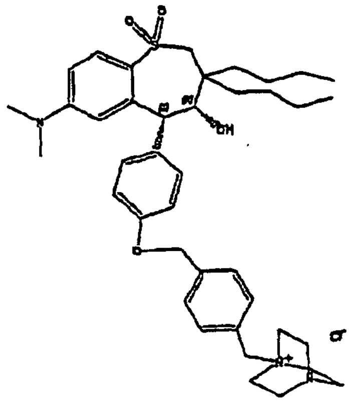

(B-5)

oder Enantiomere oder Racemate davon dargestellt wird.

**7.** Therapeutische Kombination nach Anspruch 6, worin die genannte MTP-hemmende Verbindung durch Formel M-9 dargestellt ist:

M-9

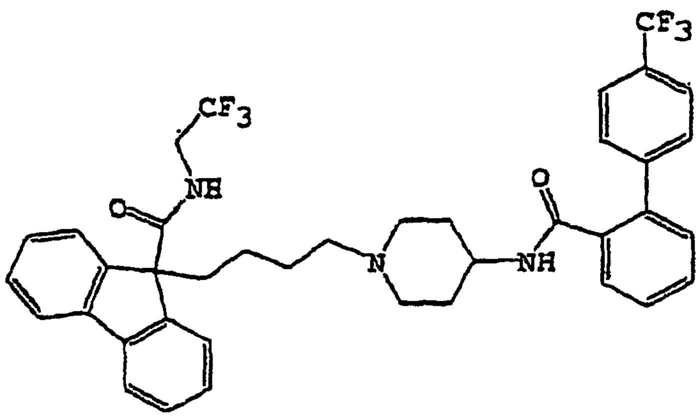

**Revendications**

**1.** Combinaison thérapeutique comprenant une première quantité d'un composé inhibant le transport iléal des acides biliaires et une seconde quantité d'un composé inhibant la protéine de transfert microsomal des triglycérides, la première quantité et la seconde quantité constituant ensemble une quantité des composés efficace en tant qu'antihyperlipidémiant, une quantité des composés efficace en tant qu'anti-athérosclérotique ou une quantité des composés efficace en tant quanti-hypercholestérolémiant.

**2.** Combinaison thérapeutique selon la revendication 1, dans laquelle le composé inhibant le transport iléal des acides biliaires a une des structures suivantes :

(1)

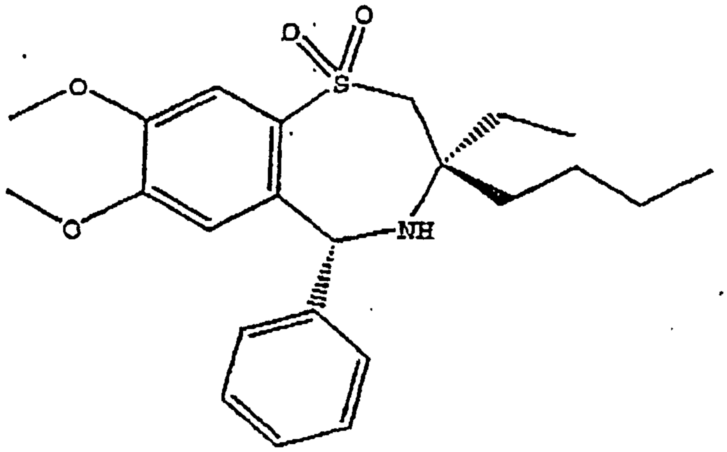

B-2

ou un énantiomère ou un racémate de celui-ci ;

(2)

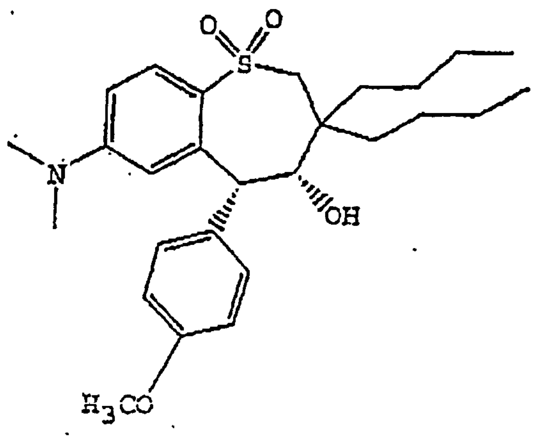

B-12

ou un énantiomère ou un racémate de celui-ci ;

(3)

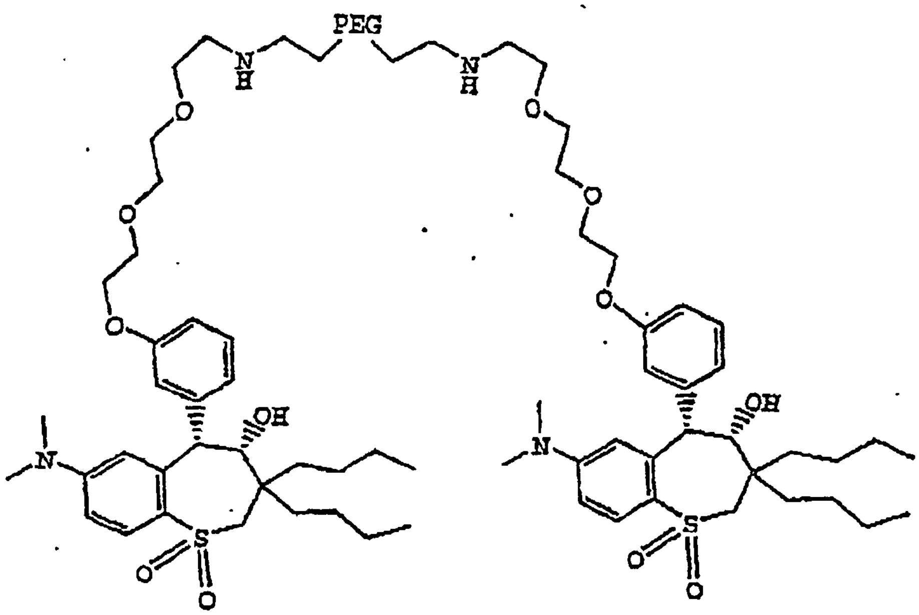

ou un énantiomère ou un racémate de celui-ci, où PEG est une chaîne polymère de polyéthylèneglycol ayant une masse moléculaire d'environ 3000 à environ 4000,

(4)

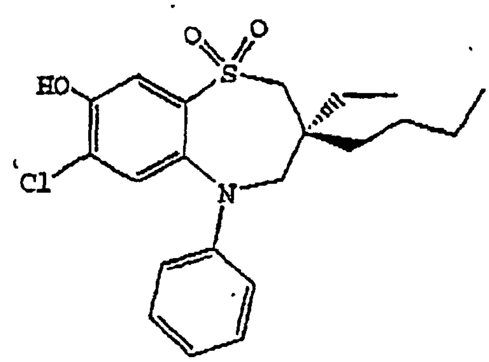

B-7

ou un énantiomère ou un racémate de celui-ci.

3. Utilisation d'une première quantité d'un composé inhibant le transport iléal des acides biliaires et d'une seconde quantité d'un composé inhibant la protéine de transfert microsomal des triglycérides, pour la fabrication d'un médicament pour la prévention ou le traitement d'une pathologie ou d'un trouble hyperlipidémique chez un mammifère, où la première quantité et la seconde quantité constituent ensemble une quantité des composés efficace en tant qu'antihyperlipidémiant, une quantité des composés efficace en tant qu'anti-athérosclérotique ou une quantité des composés efficace en tant qu'anti-hypercholestérolémiant.

4. Kit pour obtenir un effet thérapeutique chez un mammifère comprenant une quantité d'un composé inhibant le transport iléal des acides biliaires dans une première forme d'administration unitaire ; une quantité d'un composé inhibant la protéine de transfert microsomal des triglycérides dans une seconde forme d'administration unitaire ; et un moyen servant de conteneur pour contenir lesdites première et seconde formes d'administration unitaire.

5. Combinaison thérapeutique comprenant une première quantité d'un composé inhibant le transport iléal des acides

biliaires (IBAT) et une seconde quantité d'un composé inhibant la protéine de transfert microsomal des triglycérides (MTP), où ladite première quantité et ladite seconde quantité constituent ensemble une quantité efficace en tant qu'antihyperlipidémiant, une quantité efficace en tant qu'anti-athérosclérotique ou une quantité efficace en tant qu'anti-hypercholestérolémiant dudit composé inhibiteur de l'IBAT et dudit composé inhibiteur de la MTP, et où ledit composé inhibiteur de la MTP est choisi dans le groupe constitué par M-1, M-2, M-3, M-4, M-5, M-6, M-7, M-8 et M-9 représentés par

M-1

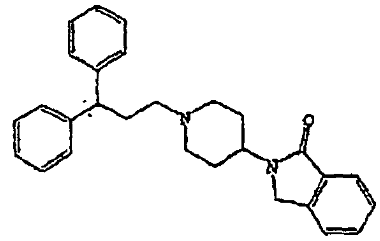

M-2

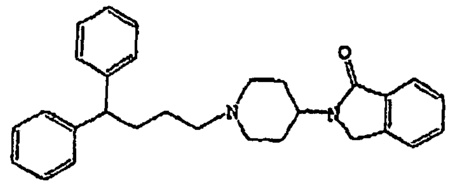

M-3

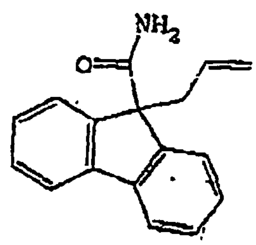

M-4

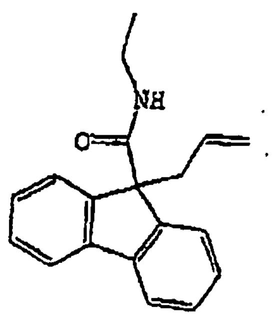

M-5

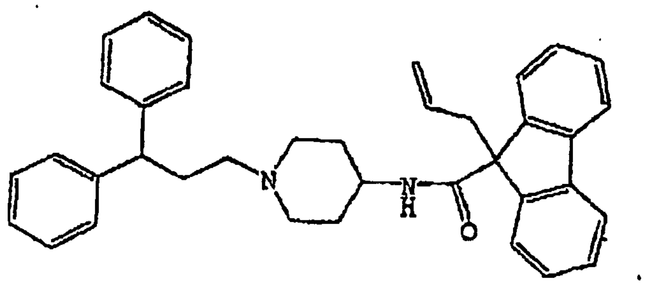

M-6

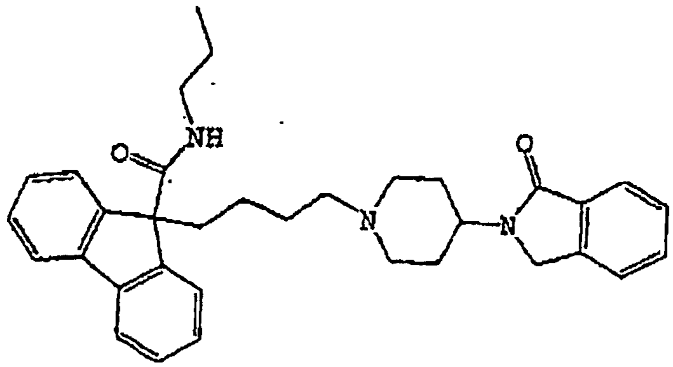

M-7

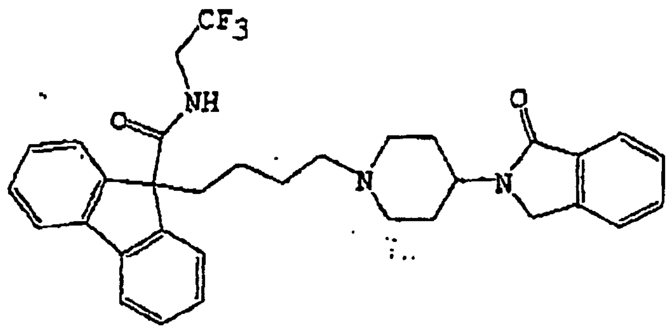

M-8

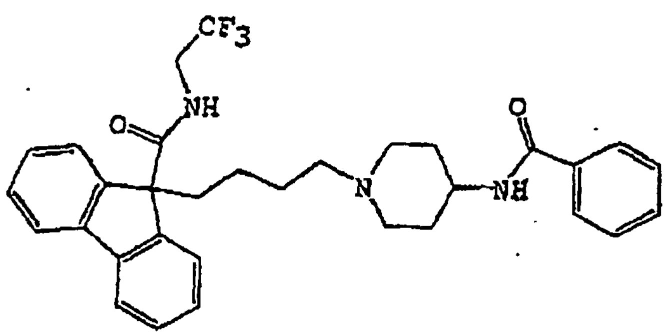

et

M-9

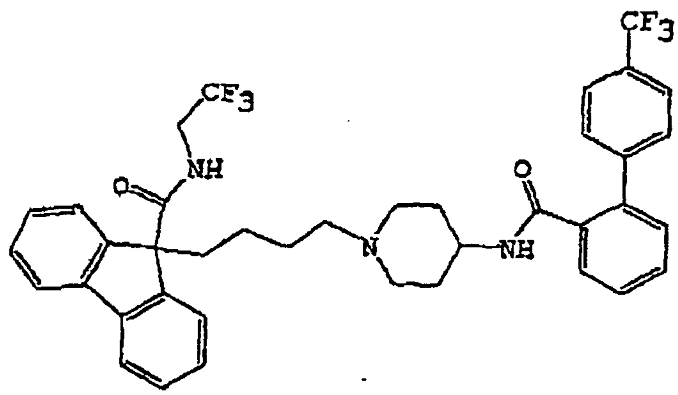

et les énantiomères, les racémates, les tautomères et les sels de ceux-ci.

6. Combinaison thérapeutique selon la revendication 5, où ledit composé inhibiteur de l'IBAT est représenté par la formule B-5 :

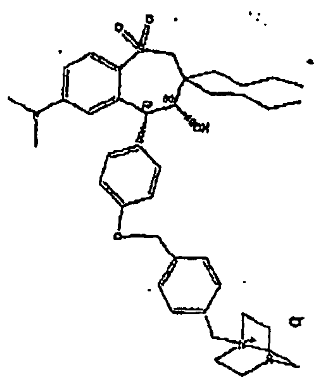

(B-5)

ou les énantiomères ou les racémates de celle-ci.

7. Combinaison thérapeutique selon la revendication 6, où ledit composé inhibiteur de la MTP est représenté par la formule M-9 :

M-9